# FASCICULE DE BREVET EUROPEEN

(11) **EP 1 000 158 B1**
(45) Date de publication et mention de la délivrance du brevet: **22.11.2006**
(21) Numéro de dépôt: 98935106.9
(22) Date de dépôt: 06.07.1998
(51) Int. Cl.: C12N 15/48, C07K 14/15, C12Q 1/68, C07K 16/10, G01N 33/569

(54) **SEQUENCES RETROVIRAUX ENDOGENES, ASSOCIEES A DES MALADIES AUTO-IMMUNES ET/OU A DES PERTURBATIONS DE LA GROSSESSE**
ENDOGENE RETROVIRALE SEQUENZEN, VERWANDTEN MIT AUTO-IMMUNE KRANKHEITEN ODER MIT SCHWANGERSCHAFTSSTÖRUNGEN
ENDOGENETIC RETROVIRAL SEQUENCES, ASSOCIATED WITH AUTOIMMUNE DISEASES OR WITH PREGNANCY DISORDERS

(30) Priorité: 07.07.1997 FR 9708815
(43) Date de publication de la demande: 17.05.2000
(73) Titulaire: BIO MERIEUX, 69280 Marcy l'Etoile (FR)
(72) Inventeur: BESEME, Frédéric, F-38090 Villefontaine (FR); BLOND, Jean-Luc, F-69005 Lyon (FR); BOUTON, Olivier, F-69340 Francheville (FR); MANDRAND, Bernard, F-69100 Villeurbanne (FR); MALLET, François, F-69100 Villeurbanne (FR); PERRON, Herve, 69005 Lyon (FR)
(74) Mandataire: Guerre, Dominique
(86) Numéro de dépôt international: PCT/FR1998/001442
(87) Numéro de publication internationale: WO 1999/002696

(56) Documents cités:
- EP-A- 0 731 168
- WO-A-93/20188
- WO-A-94/11514
- WO-A-97/06260
- PERRON H ET AL: "MOLECULAR IDENTIFICATION OF A NOVEL RETROVIRUS REPEATEDLY ISOLATED FROM PATIENTS WITH MULTIPLE SCLEROSIS" PROCEEDINGS OF THE NATIONAL ACADEMY OF SCIENCES OF USA, vol. 94, juillet 1997, pages 7583-7588, XP002062853
- MI ET AL.: "Syncytin is a captive retroviral envelope protein involved in human placental morphogenesis" NATURE, vol. 403, 17 février 2000 (2000-02-17), pages 785-789,
- X. LEE ET AL.: "Downregulation of Placental Syncytin Expression and Abnormal Protein Localization in Pre-eclampsia" PLACENTA, vol. 22, 2001, pages 808-812,
- Y. KUDO ET AL.: "Hyposxia alters expression and function of syncytin and its receptor during trophobalst cell fusion of human placental BeWo cells: implications for impaired trophoblasts syncytialisation in pre-eclampsia" BIOCHEMICA ET BIOPHYSICA ACTA, vol. 1638, 2003, pages 63-71,
- I. KNERR ET AL: "Syncytin, a novel human endogenous retroviral gene in human placenta: Evidence for its dysregulation in preeclampsia and HELLP syndrome" AM. J. OBSTET GYNECOL, vol. 186, no. 2, février 2002 (2002-02), pages 210-213,
- J. CLAUSEN: "Endogenous Retroviruses and MS: Using ERVs as disease Markers" THE INTERNATIONAL MS JOURNAL, vol. 10, 2003, pages 22-28,

### LISTAGE DE SEQUENCES

(1) INFORMATIONS GENERALES:
   (i) DEPOSANT:
      (A) NOM: BIO MERIEUX
      (B) RUE: CHEMIN DE L'ORME
      (C) VILLE: MARCY L'ETOILE
      (E) PAYS: FRANCE
      (F) CODE POSTAL: 69280
   (ii) TITRE DE L' INVENTION: MATERIEL NUCLEIQUE DE TYPE GENOMIQUE RETROVIRAL ENDOGENE, ASSOCIE A UNE MALADIE AUTO-IMMUNE ET/OU A DES PERTURBATIONS DE LA GROSSESSE ; UTILISATION EN TANT QUE MARQUEUR
   (iii) NOMBRE DE SEQUENCES: 35
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 1321 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 1:
   CAACAATCGG GATATAAACC CAGGCATTCG AGCTGGCAAC AGCAGCCCCC CTTTGGGTCC   60
   CTTCCCTTTG TATGGGAGCT GTTTTCATGC TATTTCACTC TATTAAATCT TGCAACTGCA  120
   CTCTTCTGGT CCATGTTTCT TACGGCTCGA GCTGAGCTTT TGCTCACCGT CCACCACTGC  180
   TGTTTGCCAC CACCGCAGAC CTGCCGCTGA CTCCCATCCC TCTGGATCCT GCAGGGTGTC  240
   CGCTGTGCTC CTGATCCAGC GAAGCGCCCA TTGCCGCTCC CAATTGGGCT AAAGGCTTGC  300
   CATTGTTCCT GCACGGCTAA GTGCCTGGGT TTGTTCTAAT TGAGCTGAAC ACTAGTCACT  360
   GGGTTCCATG GTTCTCTTCT GTGACCCACG GCTTCTAATA GAACTATAAC ACTTACCACA  420
   TGGCCCAAGA TTCCATTCCT TGGAATCCGT GAGGCCAAGA ACTCCAGGTC AGAGAATACG  480
   AAGCTTGCCA CCATCTTGGA AGCGGCCTGC TACCATCTTG GAAGTGGTTC ACCACCATCT  540
   TGGGAGCTCT GTGAGCAAGG ACCCCCCGGT AACATTTTGG CAACCACGAA CGGACATCCA  600
   AAGTGATGGG AAACGTTCCC CGCAAGACAA AAACGCCCCT AAGACGTATT CTGGAAAATT  660
   GGGAACAATT TGACCCTCAG ACACTAAGAA AGAAACGACT TATATTCTTC TGCAGTGCCG  720
   CCTGGCACTC CTGAGGGAAG TATAAATTAT AACACCATCT TACAGCTAGA CCTCTTTTGT  780
   AGAAAAGGCA AATGGAGTGA AGTGCCATAA GTACAAACTT TCTTTTCATT AAGAGACAAC  840
   TCACAATTAT GTAAAAAGTG TGATTTATGC CCTACAGGAA GCCTTCAGAG TCTACCTCCC  900
   TATCCCAGCA TCCCCGACTC CTTCCCCACT TAATAAGGAC CCCCCTTCAA CCCAAATGGT  960
   CCAAAAGGAG ATAGACAAAA GGGTAAACAG TGAACCAAAG AGTGCCAATA TTCCCCAATT 1020
   ATGACCCCTC CAAGCAGTGG GAGGAAGAGA ATTCGGCCCA GCCAGAGTGC ATGTGCCTTT 1080
   TTCTCTCCCA GACTTAAAGC AAATAAAAAC AGACTTAGGT AAATTCTCAG ATAACCCTGA 1140
   TGGCTATATT GGTGTTTTAC AAGGGTTAGG ACAATTCTTT GATCTGACAT GGAGAGATAT 1200
   ATATGTCACT GCTAAATCAG ACACTAACCC CAAATGAGAG AAGTGCCACC ATAACTGCAG 1260
   CCTGAGAGTT TGGCGATCTC TGGTATCTCA GTCAGGTCAA TGATAGGATG ACAACAGAGG 1320
   A 1321
(2) INFORMATIONS POUR LA SEQ ID NO: 2:
   (i) CARACTERISTIQUES DE LA SEQUENCE:
      (A) LONGUEUR: 2938 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 2:
   CAACGACGGA CATCCAAAGT GATGGGAAAC GTTCCCCGCA AGACAAAAAC GCCCCTAAGA 60
   CGTATTCTGG AGAATTGGGA CCAATTTGAC CCTCAGACAC TAAGAAAGAA ACGACTTATA 120
   TTCTTCTGCA GTGCCGCCTG GCACTCCTGA GGGAAGTATA AATTATAACA CCATCTTACA 180
   GCTAGACTTC TTTTGTAGAA AAGGCAAATG GAGTGAAGTG CCATAAGTAC AAACTTTCTT 240
   TTCATTAAGA GACAACTCAC AATTATGTAA AAAGTGTGAT TTATGCCCTA CAGGAAGCCT 300
   TCAGAGTCTA CCTCCCTATC CCAGCATCCC CGACTCCTTC CCCAACTAAT AAGGACCCCC 360
   CTTCAACCCA AATGGTCCAA AAGGAGATAG ACAAAAGGGT AAACAGTGAA CCAAAGAGTG 420
   CCAATATTCC CCAATTATGA CCCCTCCCAA GCAGTGGGAG GAAGAGATTC GGCCCAGCCA 480
   GAGTGCATGT GCTTTTTCTT CTCCCAGACT TAAAGCAAAT AAAAACAGAC TTAGGTAAAT 540
   TCTCAGATAA TCCTGATGGC TATATTGATG TTTTACAAGG GTTAGGACAA TTCTTTGATC 600
   TGACATGGAG AGATATAATG TCACTGCTAA ATCAGACACT AACCCCAAAT GAGAGAAGTG 660
   CCACCATAAC TGCAGCCTGA GAGTTTGGCG ATCTCTGGTA TCTCAGTCAG GTCAATGATA 720
   GGATGACAAC AGAGGAAAGA GATGATCCCC ACAGCCAGCA AGCAGTTCCC AGTCTASACC 780
   CTCATTGGGG ACACAGAAAT CAGTAACATG GGAGATTGGT GCTGCAGACA TTTGCTAACT 840
   TGTGTGCTAC AAGGACTAAG GAAAACTACG AAGAAAATCT ACGAATTACT CAATGATGTC 900
   CACCATAACA CAGGGGAAGG GAAGAAAATC CTACTGCCTT TCTGGAGAGA CTAAGGGAGG 960
   CATTGAGGAA GCGTGCCTCT CTGTCACCTG ACTCTTCTGA AGGCCAACTA ATCTTAAAGC 1020
   GTAAGTTTAT CACTCAGTCA GCTGCAGACA TTAGAAAAAA CTTCAAAAGT CTGCCGTAGG 1080
   CCCGGAGCAA AACTTAGAAA CCCTATTGAA CTTGGCAACY TCGGTTTTTT ATAATAGAGA 1140
   TCAGGAGGAG CAGGCGGAAC AGGACAAACG GGATTAAAAA AAAGGCCACC GCTTTAGTCA 1200
   TGACCCTCAG GCAAGTGGAC TTTGGAGGCT CTGGAAAAGG GAAAAGCTGG GCAAATTGAA 1260
   TGCCTAATAG GGCTTGCTTC CAGTGCGGTC TACAAGGACA CTTTAAAAAA GATTGTCCAA 1320
   GTAGAAGTAA GCCGCCCCTT CGTCCATGCC CCTTATTTCA AGGGAATCAC TGGAAGGCCC 1380
   ACTGCCCCAG GGGACAAAGG TCTTTTGAGT CAGAAGCCAC TAACCAGATG ATCCAGCAGC 1440
   AGGACTGAGG GTGCCTGGGG CAAGCGCCAT CCCATGCCAT CACCCTCACA GAGCCCTGGG 1500
   TATGCTTGAC CATTGAGGGC CAGGAAGGTT GTCTCCTGGA CACTGGTGCG GTCTTCTTAG 1560
   TCTTACTCTT CTGTCCCGGA CAACTGTCCT CCAGATCTGT CACTATCTGA GGGGGTCCTA 1620
   AGACGGGCAG TCACTAGATA CTTCTCCCAG CCACTAAGTT ATGACTGGGG AGCTTTATTC 1680
   TTTTCACATG CTTTTCTAAT TATGCTTGAA AGCCCCACTA CCTTGTTAGG GAGAGACATT 1740
   CTAGCAAAAG CAGGGGCCAT TATACACCTG AACATAGGAG AAGGAACACC CGTTTGTTGT 1800
   CCCCTGCTTG AGGAAGGAAT TAATCCTGAA GTCTGGGCAA CAGAAGGACA ATATGGACGA 1860
   GCAAAGAATG CCCGTCCTGT TCAAGTTAAA CTAAAGGATT CCACTTCCTT TCCCTACCAA 1920
   AGGCAGTACC CCCTCAGACC CAAGGCCCAA CAAGGATTCC AAAAGATTGT TAAGGACTTA 1980
   AAAGCCCAAG GCTTAGTAAA ACCATGCATA ACTCCCTGCA GTAATTCCGT AGTGGATTGA 2040
   GGAGGCACAG AAACCCAGTG GACAGTGGAG GGTTAGTGCA AGATCTCAGG ATTATCAATG 2100
   GAGGCCGTTG TCCTTTTATA CCCAGCTGTA CCTAGCCCTT ATACTGTGCT TTCCCAAATA 2160
   CCAGAGGAAG CAGAGTGGTT TACACTCCTG GACCTTAAGG ATGCCTTCTT CTGCATCCCT 2220
   GTACATCCTG ACTCTCAATT CTTGTTTGCC TTTGAAGATA CTTCAAACCC AACATCTCAA 2280
   CTCACCTGGA CTGTTTTACC CCAAGGGTTC AGGGATAGCC CCCATCTATT TGGCCAGGCA 2340
   TTAGCCCAAG ACTTGAGCCA ATCCTCATAC CTGGACACTT GTCCTTCGGT AGGTGGATGA 2400
   TTTACTTTTG GCCGCCCATT CAGAAACCTT GTGCCATCAA GCCACCCAAG CGCTCTTCAA 2460
   TTTCCTCGCT ACCTGTGGCT ACATGGTTTC CAAACCAAAG GCTCAACTCT GCTCACAGCA 2520
   GGTTACTTAG GGCTAAAATT ATCCAAAGGC ACCAGGGCCC TCAGTGAGGA ACACATCCAG 2580
   CCTATACTGG CTTATCCTCA TCCCAAAACC CTAAAGCAAC TAAGGGGATT CCTTGGCGTA 2640
   ATAGGTTTCT GCCGAAAATG GATTCCCAGG TTTGGCGAAA TAGCCAGGTC ATTAAATACA 2700
   CTAATTAAGG AAACTCAGAA AGCCAATACC CATTTAGTAA GATGGACAAC TGAAGTAGAA 2760
   GTGGCTTTCC AGGCCCTAAC CCAAGCCCCA GTGTTAAGTT TGCCAACAGG GCAAGACTTT 2820
   TCTTCATATG TCACAGAAAA AACAGGAATA GCTCTAGGAG TCCTTACACA GATCCGAGGG 2880
   ATGAGCTTGC AACCTGTGGC GTACCTGACT AAGGAAATTG ATGTAGTGGC AAAGGGTT 2938
(2) INFORMATIONS POUR LA SEQ ID NO: 3:
   (i) CARACTERISTIQUES DE LA SEQUENCE:
      (A) LONGUEUR: 1422 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 3:
   TCAGGGATAG CCCCCATCTA TTTGGCCAGG CATTAGCCCA AGACTTGAGT CAGTTATCAT 60
   ACCTGGACAC TCTTGTCCTT CAGTATGTGG ATGATTTACT TTTAGCTGCC TGTTCAGAAA 120
   CCTTGTGCCA TCAAGCCACC CAAGCACTCT TAAATTTCCT CGCCACCTGT GGCTACAAGG 180
   TTTCCAAAGA GAAGCTCAGC TCTGCTCACA GCAGGTTAAA TACTTAGGAC TAAGATTATC 240
   CAAAGGCACC AAGGCCCTCA GTGAGGAATG TATCCAGCCT ATACTGGCTT ATCCTCATCT 300
   CAAAACCCTA AAGCAACTAA GAGAGTTCCT TGGCATAACA GGCTTCTGCC GAATATGGAT 360
   TCCCCAGGTA TGGCAAAATA GCCAGGCCAT TATATACAGT AATTAAGGAA ACTCAGAAAG 420
   CCAATACCCA TTTAATAAGA TGGATACCTG AAGCCAAAGT GGCTTTCCAG GCCCCTAAAG 480
   AAGGCCTTAA ACCCAAGTCC CAGTGTTAAG CTTGCCAACG GGGCAAGACT TTTCTTTATA 540
   CATCACAGAA AAAAACAGAA ACAGCTCTGG GAGTCCTTAC ACAGGTCCAA GGGACGAGCT 600
   TGCAACCCAT GGCATACCTG AGTAAGGAAA CTGATGTAGT GGCAAAGGGT TGGCTTCATT 660
   GTTTATGGGT AGTGGTGGCA GTAGCAGTTG TAGTATCTGA AGCAGTTAAA ATAATACAGG 720
   GGAGAGATCT TACTGTGTGG ACATCTCATG AGGTGAACAG CATACTCACT GCTAAAGGAG 780
   ACTTGTGGCT GTCAGACAAC CGTTTACTTA AATATCAGGC TCTATTACTT GAAAGGCCAG 840
   TGCTGCAACT GTGCACTTGT GCAACTCTTA ACCCAGTCNC ATTTCTTCCA GACAATGAAG 900
   ATAGAATATA ACTGTCAACA AATAATTTCT CAAACCTATG CCACTCGAGG GGACCTTCTA 960
   GAAGTTCCCT TGACTGATCC TGACCTTCAA CTTGTATACT GATGGAAGTT CCTTTGTAGA 1020
   AAAAGGACTT CAAAAGCGGG GTATGCAGTG GTCAGTGATA ATGGAATATT TGAAAGTATC 1080
   CCCTCACTCC AGGAACTAGT GCTTAGCTGG CAGAACTAAT AGCCTTCATT GGGGCACTAG 1140
   AATTAGGAGA AGGAAAAAGG GTAAATATAT ATACAGACTC TGAGTATGCT CACCTAGTCN 1200
   TCCATGCCCA TGAGGCAATA TGCAGAGAAA GGGAATTCCT AACTTCCGAG GGAACACCTA 1260
   TCACACATCA GGAAGCCATT AGGAGATTAT TACTGGCAGT ACAGAAACCT AAAGAGGTGG 1320
   AAGTCTTACA CTGCTGGGGT CATCAGAAAG GAAAGAAAAG GGAAATAGAA GGGAATTGCC 1380
   AAGCAGATAT TGAAGCAAAA AGAGCTGCAA GGCAGGACCC TC 1422
(2) INFORMATIONS POUR LA SEQ ID NO: 4:
   (i) CARACTERISTIQUES DE LA SEQUENCE:
      (A) LONGUEUR: 2006 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 4:
   ATGCAGTGGT CAGTGATAAT GGAATACTTG AAAGTAATCC CCTCACTCCA GGAACTAGTG 60
   CTCAGCTAGC AGAACTAATA GCCCTCACTT GGGCACTAGA ATTAGGAGAA GAAAAAAGGG 120
   CAAATATATA TACAGACTCT AAATATGCTT ACCTAGTCCT CCATGCCCAT GCAGCAATAT 180
   GGAAAGAAAG GGAATTCCTA ACTTCTGAGA GAACACCTAT CAAACATCAG GAAGCCATTA 240
   GGAAATTATT ATTGGCTGTA CAGAAACCTA AAGAGGTGGC AGTCTTACAC TGCCGGGGTC 300
   ATCANAAAGG AAAGGAAAGG GAAAATACTT TTGCCTGCAA CTATCCAATG GAAATTACTT 360
   AAAACCCTTC ATCAAACCTT TCACTTAGGC ATCGATAGCA CCCATCAAAT GGCCAAATCA 420
   TTATTTACTG GACCAGGCCT TTTCAAAACT ATCAAGCAAA TATTCAGGGC CTGTGAATTG 480
   TGCCAAAAAA ATAATCCCCT GCCTCATCGC CAAGCTCCTT CAGGAAAACA AAAAACAGGC 540
   CATTACCCTG AAAAAAACTG GCAACTGATT TTACCCACAA GCCCAAACCT CAGGGATTTC 600
   AGTATCTACT AGTCTGGGTA AATACTTTCA CGGGTTGGGC AAAGGCCTTC CCCTGTAGGA 660
   CAGAAAAGGC CCAAGAGGTA ATAAAGGCAC TAGTTCATGA AATAATTCCC AGATTCGGAC 720
   TTCCCCGAGG CTTACAGAGT GACAATAGCC CTGCTTTCCA GGCCACAGTA ACCCAGGGAG 780
   TATCCCAGGC GTTAGGTATA CGATATCACT TACACTGCGC CTGAAGGCCA CAGTCCTCAG 840
   GGAAGGTCGA GAAAATGAAT GAAATACTCA AAGGACATCT AAAAAAGCAA ACCCAGGAAA 900
   CCCACCTCAC ATGGCCTGCT CTGTTGCCTA TAGCCTTAAA AAGAATCTGC AACTTTCCCC 960
   AAAAAGCAGG ACTTAGCCCA TACGAAATGC TGTATGGAAG GCCCTTCATA ACCAATGACC 1020
   TTGTGCTTGA CCCAAGACAG CCAACTTAGT TGCAGACATC ACCTCCTTAG CCAAATATCA 1080
   ACAAGTTCTT AAAACATTAC AAGGAACCTA TCCCTGAGAA GAGGGAAAAG AACTATTCCA 1140
   CCCTTGTGAC ATGGTATTAG TCAAGTCCCT TCTCTCTAAT TCCCCATCCC TAGATACATC 1200
   CTGGGAAGGA CCCTACCCAG TCATTTTATT TACCCCAACT GCGGTTAAAG TGGCTGGAGT 1260
   GGTCTTGGAT ACATCACACT TGAGTCAAAT CCTGGATACT GCCAAAGGAA CCTGAAAATC 1320
   CAGGAGACAA CGCTAGCTAT TCCTGTGAAC CTCTAGAGGA TTTGCGCCTG CTCTTCAAAC 1380
   AACAACCAGG AGGAAAGTAA CTAAAATCAT AAATCCCCCA TGGCCCTCCC TTATCATATT 1440
   TTTCTCTTTA CTGTTCTTTT ACCCTCTTTC ACTCTCACTG CACCCCCTCC ATGCCGCTGT 1500
   ATGACCAGTA GCTCCCCTTA CCAAGAGTTT CTATGGAGAA TGCAGCGTCC CGGAAATATT 1560
   GATGCCCCAT CGTATAGGAG TCTTTCTAAG GGAACCCCCA CCTTCACTGC CCACACCCAT 1620
   ATGCCCCGCA ACTGCTATCA CTCTGCCACT CTTTGCATGC ATGCAAATAC TCATTATTGG 1680
   ACAGGAAAAA TGATTAATCC TAGTTGTCCT GGAGGACTTG GAGTCACTGT CTGTTGGACT 1740
   TACTTCACCC AAACTGGTAT GTCTGATGGG GGTGGAGTTC AAGATCAGGC AAGAGAAAAA 1800
   CATGTAAAAG AAGTAATCTC CCAACTCACC CGGGTACATG GCACCTCTAG CCCTACAAAG 1860
   GACTAGATCT CTCAAAACTA CATGAAACCC TCCGTACCCA TACTCGCCTG GTAAGCCTAT 1920
   TTAATACCAC CCTCACTGGG CTCCATGAGG TCTCGGCCCA AAACCCTACT AACTGTTGGA 1980
   TATGCCTCCC CCTGAACTTC AAGCCA 2006
(2) INFORMATIONS POUR LA SEQ ID NO: 5:
   (i) CARACTERISTIQUES DE LA SEQUENCE:
      (A) LONGUEUR: 1948 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 5:
   ACTGCACTCT TCTGGTCCAT GTTTCTTACG GCTCGAGCTG AGCTTTTGCT CACCGTCCAC 60
   CACTGCTGTT TGCCACCACC GCANACCTGC CGCTGACTCC CATCCCTCTG GATCCTGCAG 120
   GGTGTCCGCT GTGCTCCTGA TCCAGCGAGG CGCCCATTGC CGCTCCCAAT TGGGCTAAAG 180
   GCTTGCCATT GTNCCTGCAC GGCTAAGTGC CTGGGTTTGT TCTAATTGAG CTGAACACTA 240
   NTCACTGGGT TCCATGGTTC TCTTCTGTGA CCCACGGCTT CTAATAGAAC TATAACACTT 300
   ACCACATGGC CCAAGATTCC ATTCCTTGGA ATCCGTGAGG GCAAGAACTC CAGGTCAGAG 360
   AATACGAGGC TTGCCACCAT CTTGGAAGCG GCCTGCTACC ATCTTGGAAG TGGTTCACCA 420
   CCATCTTGGG AGCTCTGTGA GCAAGGACCC CCCGGTAACA TTTTGGCAAC CACGAACGGA 480
   CATCCAAAGT GATACATCCT GGGAAGGACC CTACCCAGTC ATTTTATCTA CCCCAACTGC 540
   GGTTAAAGTG GCTGGAGTGG AGTCTTGGAT ACATCACACT TGAGTCAAAT CCTGGATACT 600
   GCCAAAGGAA CCTGAAAATC CAGGAGACAA CGCTAGCTAT TCCTGTGAAC CTCTAGAGGA 660
   TTTGCGCCTG CTCTTCAAAC AACAACCAGG AGGAAAGTAA CTAAAATCAT AAATCCCCAT 720
   GGCCCTCCCT TATCATATTT TTCTCTTTAC TGTTGTTTCA CCCTCTTTCA CTCTCACTGC 780
   ACCCCCTCCA TGCCGCTGTA TGACCAGTAG CTCCCCTTAC CAAGAGTTTC TATGGAGAAT 840
   GCAGCGTCCC GGAAATATTG ATGCCCCATC GTATAGGAGT CTTTGTAAGG GAACCCCCAC 900
   CTTCACTGCC CACACCCATA TGCCCCGCAA CTGCTATCAC TCTGCCACTC TTTGCATGCA 960
   TGCAAATACT CATTATTGGA CAGGAAAAAT GATTAATCCT AGTTGTCCTG GAGGACTTGG 1020
   AGTCACTGTC TGTTGGACTT ACTTCACCCA AACTGGTATG TCTGATGGGG GTGGAGTTCA 1080
   AGATCAGGCA AGAGAAAAAC ATGTAAAAGA AGTAATCTCC CAACTCACCC GGGTACATGG 1140
   CACCTCTAGC CCCTACAAAG GACTAGATCT CTCAAAACTA CATGAAACCC TCCGTACCCA 1200
   TACTCGCCTG GTAAGCCTAT TTAATACCAC CCTCACTGGG CTCCATGAGG TCTCGGCCCA 1260
   AAACCCTACT AACTGTTGGA TATGCCTCCC CCTGAACTTC AGGCCATATG TTTCAATCCC 1320
   TGTACCTGAA CAATGGAACA ACTTCAGCAC AGAAATAAAC ACCACTTCCG TTTTAGTAGG 1380
   ACCTCTTGTT TCCAATCTGG AAATAACCCA TACCTCAAAC CTCACCTGTG TAAAATTTAG 1440
   CAATACTACA TACACAACCA ACTCCCAATG CATCAGGTGG GTAACTCCTC CCACACAAAT 1500
   AGTCTGCCTA CCCTCAGGAA TATTTTTTGT CTGTGGTACC TCAGCCTATC GTTGTTTGAA 1560
   TGGCTCTTCA GAATCTATGT GCTTCCTCTC ATTCTTAGTG CCCCCTATGG CCATCTACAC 1620
   TGAACAAGAT TTATACAGTT ATGTCATATC TAAGCCCCGC AACAAAAGAG TACCCATTCT 1680
   TCCTTTTGTT ATAGGAGCAG GAGTGCTAGG TGCACTAGGT ACTGGCATTG GCGGTATCAC 1740
   AACCTCTACT CAGTTCTACT ACAAACTATC TCAAGAACTA AATGGGGACA TGGAACGGGT 1800
   CGCCGACTCC CTGGTCACCT TGCAAGATCA ACTTAACTCC CTAGCAGCAG TAGTCCTTCA 1860
   AAATCGAAGA GCTTTAGACT TGCTAACCGC TGAAAGAGGG GGAACCTGTT TATTTTTAGG 1920
   GGAAGAATGC TGTTATTATG TTAATCAA                                   1948
(2) INFORMATIONS POUR LA SEQ ID NO: 6:
   (i) CARACTERISTIQUES DE LA SEQUENCE:
      (A) LONGUEUR: 1136 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 6:
   CCATGGCCAT CTACACTGAA CAAGATTTAT ACAGTTATGT CATATCTAAG CCCCGCAACA   60
   AAAGAGTACC CATTCTTCCT TTTGTTATAG GAGCAGGAGT GCTAGGTGCA CTAGGTACTG  120
   GCATTGGCGG TATCACAACC TCTACTCAGT TCTACTACAA ACTATCTCAA GAACTAAATG  180
   GGGACATGGA ACGGGTCGCC GACTCCCTGG TCACCTTGCA AGATCAACTT AACTCCCTAG  240
   CAGCAGTAGT CCTTCAAAAT CGAAGAGCTT TAGACTCGCT AACCGCTGAA AGAGGGGGAA  300
   CCTGTTTATT TTTAGGGGAA GAATGCTGTT ATTATGTTAA TCAATCCGGA ATCGTCACTG  360
   AGAAAGTTAA AGAAATTCGA GATCGAATAC AACGTAGAGC AGAAGAGCTT CGAAACACTG  420
   GACCCTGGGG CCTCCTCAGC CAATGGATGC CCTGGATTCT CCCCTTCTTA GGACCTCTAG  480
   CAGCTATAAT ATTGCTACTC CTCTTTGGAC CCTGTATCTT TAACCTCCTT GTTAACTTTG  540
   TCTCTTCCAG AATCGAAGCT GTAAAACTAC AAATGGAGCC CAAGATGCAG TCCAAGACTA  600
   AGATCTACCG CAGACCCCTG GACCGGCCTG CTAGCCCACG ATCTGATGTT AATGACATCA  660
   AAGGCACCCC TCCTGAGGAA ATCTCAGCTG CACAACCTCT ACTACGCCCC AATTCAGCAG  720
   GAAGCAGTTA GAGCGGTCGT CGGCCAACCT CCCCAACAGC ACTTAGGTTT TCCTGTTGAG  780
   ATGGGGGACT GAGAGACAGG ACTAGCTGGA TTTCCTAGGC TGACTAAGAA TCCCTAAGCC  840
   TAGCTGGGAA GGTGACCACA TCCACCTTTA AACACGGGGC TTGCAACTTA GTTCACACCT 900
   GACCAATCAG AGAGCTCACT AAAATGCTAA TTAGGCAAAG ACAGGAGGTA AAGAAATAGC 960
   CAATCATCTA TTGCATGAGA GCACAGCAGG AGGGACAATG ATCGGGATAT AAACCCAAGT 1020
   CTTCGAGCCG GCAACGGCAA CCCCCTTTGG GTCCCCTCCC TTTGTATGGG AGCTCTGTTT 1080
   TCATGCTATT TCACTCTATT AAATCTTGCA GCTGCGAAAA AAAAAAAAAA AAAAAA 1136
(2) INFORMATIONS POUR LA SEQ ID NO: 7:
   (i) CARACTERISTIQUES DE LA SEQUENCE:
      (A) LONGUEUR: 2782 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 7
   ATGGGAGCTG TTTTCATGCT ATTTCACTCT ATTAAATCTT GCAACTGCAC TCTTCTGGTC 60
   CATGTTTCTT ACGGCTCGAG CTGAGCTTTT GCTCACCGTC CACCACTGCT GTTTGCCACC 120
   ACCGCAGACC TGCCGCTGAC TCCCATCCCT CTGGATCCTG CAGGGTGTCC GCTGTGCTCC 180
   TGATCCAGCG AAGCGCCCAT TGCCGCTCCC AATTGGGCTA AAGGCTTGCC ATTGTTCCTG 240
   CACGGCTAAG TGCCTGGGTT TGTTCTAATT GAGCTGAACA CTAGTCACTG GGTTCCATGG 300
   TTCTCTTCTG TGACCCACGG CTTCTAATAG AACTATAACA CTTACCACAT GGCCCAAGAT 360
   TCCATTCCTT GGAATCCGTG AGGCCAACGA ACTCCAGGTC AGAGAATACG AAGCTTGCCA 420
   CCATCTTGGA AGCGGCCTGC TACCATCTTG GAAGTGGTTC ACCACCATCT TGGGAGCTCT 480
   GTGAGCAAGG ACCCCCCGGT GACATTTTGG CGACCACCAA CGGACATCCC AAGTGATACA 540
   TCCTGGGAAG GACCCTACCC AGTCATTTTA TCTACCCCAA CTGCGGTTAA AGTGGCTGGA 600
   GTGGAGTCTT GGATACATCA CACTTGAGTC AAATCCTGGA TACTGCCAAA GGAACCTGAA 660
   AATCCAGGAG ACAACGCTAG CTATTCCTGT GAACCTCTAG AGGATTTGCG CCTGCTCTTC 720
   AAACAACAAC CAGGAGGAAA GTAACTAAAA TCATAAATCC CCATGGGCCT CCCTTATCAT 780
   ATTTTTCTCT GTAGTGTTCT TTCACCCTGT TTCACTCTCA CTGCACCCCC TCCATGCCGC 840
   TGTATGACCA GTAGCTCCCC TCACCCAGAG TTTCTATGGA GAATGCAGCG TCCCGGAAAT 900
   ATTGATGCCC CATCGTATAG GAGTCTTTCT AAGGGAACCC CCACCTTCAC TGCCCACACC 960
   CATATGCCCC GCAACTGCTA TCACTCTGCC ACTCTTTGCA TGCATGCAAA TACTCATTAT 1020
   TGGACAGGAA AAATGATTAA TCCTAGTTGT CCTGGAGGAC TTGGAGTCAC TGTCTGTTGG 1080
   ACTTACTTCA CCCAAACTGG TATGTCTGAT GGGGGTGGAG TTCAAGATCA GGCAAGAGAA 1140
   AAACATGTAA AAGAAGTAAT CTCCCAACTC ACCGGGGTAC ATGGCACCTC TAGCCCCTAC 1200
   AAAGGACTAG ATCTCTCAAA ACTACATGAA ACCCTCCGTA CCCATACTCG CCTGGTAAGC 1260
   CTATTTAATA CCACCCTCAC TGGGCTCCAT GAGGTCTCGG CCCAAAACCC TACTAACTGT 1320
   TGGATATGCC TCCCCCTGAA CTTCAGGCCA TATGTTTCAA TCCCTGTACC TGAACAATGG 1380
   AACAACTTCA GCACAGAAAT AAACACCACT TCCGTTTTAG TAGGACCTCT TGTTTCCAAT 1440
   GTGGAAATAA CCCATACCTC AAACCTCACC TGTGTAAAAT TTAGCAATAC TACATACACA 1500
   ACCAACTCCC AATGCATCAG GTGGGTAACT CCTCCCACAC AAATAGTCTG CCTACCCTCA 1560
   GGAATATTTT TTGTCTGTGG TACCTCAGCC TATCGTTGTT TGAATGGCTC TTCAGAATCT 1620
   ATGTGCTTCC TCTCATTCTT AGTGCCCCCT ATGACCATCT ACACTGAACA AGATTTATAC 1680
   AGTTATGTCA TATCTAAGCC CCGCAACAAA AGAGTACCCA TTCTTCCTTT TGTTATAGGA 1740
   GCAGGAGTGC TAGGTGCACT AGGTACTGGC ATTGGCGGTA TCACAACCTC TACTCAGTTC 1800
   TACTACAAAC TATCTCAAGA ACTAAATGGG GACATGGAAC GGGTCGCCGA CTCCCTGGTC 1860
   ACCTTGCAAG ATCAACTTAA CTCCCTAGCA GCAGTAGTCC TTCGAAATCG AAGAGCTTTA 1920
   GACTTGCTAA CCGCTGAGAG AGGGGGAACC TGTTTATTTT TAGGGGAAGA ATGCTGTTAT 1980
   TATGTTAATC AATCCGGAAT CGTCACTGAG AAAGTTGAAG AAATTCCAGA TCGAATACAA 2040
   CGTATAGCAG AGGAGCTTCG AAACACTGGA CCCTGGGGCC TCCTCAGCCG ATGGATGCCC 2100
   TGGATTCTCC CCTTCTTAGG ACCTCTAGCA GCTATAATAT TGCTACTCCT CTTTGGACCC 2160
   TGTATCTTTG ACCTCCTTGT TAACTTTGTC TCTTCCAGAA TCGAAGCTGT GAAACTACAA 2220
   ATGGAGCCCA AGATGCAGTC CAAGACTAAG ATCTACCGCA GACCCCTGGA CCGGCCTGCT 2280
   AGCCCACGAT CTGATGTTAA TGACATCAAA GGCACCCCTC CTGAGGAAAT CTCAGCTGCA 2340
   CAACCTCTAC TACGCCCCAA TTCAGCAGGA AGCAGTTAGA GCGGTGGTCG GCCAACCTCC 2400
   CCAACAGCAC TTAGGTTTTC CTGTTGAGAT GGGGGACTGA GAGACAGGAC TAGCTGGATT 2460
   TCCTAGGCTG ACTAAGAATC CTTAAGCCTA GGTGGGAAGG TGACCACATC CACCTTTAAA 2520
   CACGGGGCTT GCAACTTAGC TCACACCTGA CCAATCAGAG AGCTCACTAA AATGCTAATT 2580
   AGGCAAAGAC AGGAGGTAAA GAAATAGCCA ATCATTTATT GCCTGAGAGC ACAGCAGGAG 2640
   GGACAATGAT CGGGATATAA ACCCAAGTTT TCGAGCCGGC AACGGCAACC CCCTTTGGGT 2700
   CCCCTCCCTT TGTATGGGAG CTCTGTTTTC ATGCTATTTC ACTCTATTAA ATCTTGCAAC 2760
   TGCAAAAAAA AAAAAAAAAA AA                                           2782
(2) INFORMATIONS POUR LA SEQ ID NO: 8:
   (i) CARACTERISTIQUES DE LA SEQUENCE:
      (A) LONGUEUR: 666 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 8:
   TGTCCGCTGT GCTCCTGATC CAGCGAGGCG CCCATTGCCG CTCCCAATTG GGCTAAAGGC   60
   TTGCCATTGT TCCTGCACGG CTAAGTGCCT GGGTTTGTTC TAATTGAGCT GAACACTANT  120
   CACTGGGTTC CATGGTTCTC TTCTGTGACC CACGGCTTCT AATATAACTA TAACACTTAC  180
   CACATGGCCC AAGATTCCAT TCCTTGGAAT CCGTGAGGCC AAGAACTCCA GGTCAGAGAA  240
   TACGAGGCTT GCCACCATCT TGGAAGCGGC CTGCTACCAT CTTGGAAGTG GTTCACCACC  300
   ATCTTGGGAG CTCTGTGAGC AAGGACCCCC CGGTAACATT TTGGCAACCA CGAACGGACA   360
   TCCAAAGTGA ATCGAAGCTG TAAAACTACA AATGGAGCCC AAGATGCAGT CCAAGACTAA   420
   GATCTACCGC AGACCCCTGG ACCGGCCTGC TAGCCCACGA TCTGATGTTA ATGACATCAA   480
   AGGCACCCCT CCTGAGGAAA TCTCAGCTGC ACAACCTCTA CTACGCCCCA ATTCAGCAGG   540
   AAGCAGTTAG AGCGGTCGTC GGCCAACCTC CCCAACAGCA CTTAGGTTTT CCTGTTGAGA   600
   TGGGGGACTG AGAGACAGGA CTAGCTGGAT TTCCTAGGCT GACTAAGAAT CCCTAAGCCT   660
   AGCTGG                                                              666
(2) INFORMATIONS POUR LA SEQ ID NO: 9:
   (i) CARACTERISTIQUES DE LA SEQUENCE:
      (A) LONGUEUR: 3372 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 9:
   GACTTCCCAA ATACCAGAGG AAGCAGAGTG GTTTACAGTC CTGGACCTTC AGGATGCCTT    60
   CTTCTGCATC CCTGTACATC CTGACTCTCA ATTCTTGTTT GCCTTTGAAG ATACTTCAAA   120
   CCCAGCATCT CAACTCACCT GGACTATTTT ACCCCAAGGG TTCAGGGATA GTCCCCATCT   180
   ATTTGGCCAG GCATTAGCCC AAGACTTGAG CCAATCCTCA TACCTGGACA CTTGTCCTTC   240
   GGTAGGTGGA TGATTTACTT TTGGCCGCCC ATTCAGAAAC CTTGTGCCAT CAAGCCACCC 300
   AAGCGCTCTT CAATTTCCTC GCTACCTGTG GCTACATGGT TTCCAAACCA AAGGCTCAAC 360
   TCTGCTCACA GCAGGTTACT TAGGGCTAAA ATTATCCAAA GGCACCAGGG CCCTCAGTGA 420
   GGAACACATC CAGCCTATAC TGGCTTATCC TCATCCCAAA ACCCTAAAGC AACTAAGGGG 480
   ATTCCTTGGC GTAATAGGTT TCTGCCGAAA ATGGATTCCC AGGTATGGCG AAATAGCCAG 540
   GTCATTAAAT ACACTAATTA AGGAAACTCA GAAAGCCAAT ACCCATTTAG TAAGATGGAC 600
   AACTGAAGTA GAAGTGGCTT TCCAGGCCCT AACCCAAGCC CCAGTGTTAA GTTTGCCAAC 660
   AGGGCAAGAC TTTTGTTCAT ATGTCACAGA AAAAACAGGA ATAGCTCTAG GAGTCCTTAC 720
   ACAGATCCGA GGGATGAGCT TGCAACCTGT GGCACACCTG ACTAAGGAAA TTGATGTAGT 780
   GGCAAAGGGT TGACCTCATT GTTTACGGGT AGTGGTGGCA GTAGCAGTCT TAGTATCTGA 840
   AGCAGTTAAA ATAATACAGG GAAGAGATCT TACTGTGTGG ACATCTCATG ATGTGAATGG 900
   CATACTCACT GCTAAAGGAG ACTTGTGGCT GTCAGACAAC TGTTTACTTA AATGTCAGGC 960
   TCTATTACTT GAAGGGCCAG TGCTGCGACT GTGCACTTGT GCAACTCTTA ACCCAGCCAC 1020
   ATTTCTTCCA GACAATGAAG AAAAGATAAA ACATAACTGT CAACAAGTAA TTTCTCAAAC 1080
   CTATGCCACT CGAGGGGACC TTTTAGAGGT TCCTTTGACT GATCCCGACC TCAACTTGTA 1140
   TACTGATGGA AGTTCCTTTG TAGAAAAAGG ACTTCGAAAA GTGGGGTATG CAGTGGTCAG 1200
   TGATAATGGA ATACTTGAAA GTAATCCCCT CACTCCAGGA ACTAGTGCTC AGCTAGCAGA 1260
   ACTAATAGCC CTCACTTGGG CACTAGAATT AGGAGAAGAA AAAAGGGCAA ATATAATACA 1320
   GACTCTAAAT ATGCTTACCT AGTCCTCCAT GCCCATGCAG CAATATGGAA AGAAAGGGAA 1380
   TTCCTAACTT CTGAGAGAAC ACCTATCAAA CATCAGGAAG CCATTAGGAA ATTATTATTG 1440
   GCTGTACAGA AACCTAGAGA GGTGGCAGTC TTACACTGCC GGGGTCATCA CAAAGGAAAG 1500
   GAAAGGGAAA TACAAGAGAA CTGCCAAGCA TATATTGAAG CCAAAAGAGC TGCAAGGCAG 1560
   GACCCTCCAT TAGAAATGCT TATTAAACTT CCCTTAGTAT AGGGTAATCC CTTCCGGGAA 1620
   ACCAAGCCCC AGTACTCAGC AGGAGAAACA GAATGGGGAA CCTCACGAGG CAGTTTTCTC 1680
   CCCTCGGGAC GGTTAGCCAC TGAAGAAGGG AAAATACTTT TGCCTGCAAC TATCCAATGG 1740
   AAATTACTTA AAACCCTTCA TCAAACCTTT CACTTAGGCA TCGATAGCAC CCATCAGATG 1800
   GCCAAATCAT TATTTACTGG ACCAGGCCTT TTCAAAACTA TCAAGCAGAT AGTCAGGGCC 1860
   TGTGAAGTGT GCCAGAGAAA TAATCCCCTG CCTTATCGCC AAGCTCCTTC AGGAGAACAA 1920
   AGAACAGGCC ATTACCCTGG AGAAGACTGG CAACTGATTT TACCCACAAG CCCAAACCTC 1980
   AGGGATTTCA GTATCTACTA GTCTGGGTAG ATACTTTCAC GGGTTGGGCA GAGGCCTTCC 2040
   CCTGTAGGAC AGAAAAGGCC CAAGAGGTAA TAAAGGCACT AGTTCATGAA ATAATTCCCA 2100
   GATTCGGACT TCCCCGAGGC TTACAGAGTG ACAATAGCCC TGCTTTCCAG GCCACAGTAA 2160
   CCCAGGGAGT ATCCCAGGCG TTAGGTATAC GATATCACTT ACACTGCGCC TGAAGGCCAC 2220
   AGTCCTCAGG GAAGGTCGAG AAAATGAATG AAACACTCAA AGGACATCTA AAAAAGCAAA 2280
   CCCAGGAAAC CCACCTCACA TGGCCTGTTC TGTTGCCTAT AGCCTTAAAA AGAATCTGCA 2340
   ACTTTCCCCA AAAAGCAGGA CTTAGCCCAT ACGAAATGCT GTATGGAAGG CCCTTCATAA 2400
   CCAATGACCT TGTGCTTGAC CCAAGACAGC CAACTTAGTT GCAGACATCA CCTCCTTAGC 2460
   CAAATATCAA CAAGTTCTTA AAACATTACA AGGAACCTAT CCCTGAGAAG AGGAAAAGAA 2520
   TATTCCACCC AAGTGACATG GTATTAGTCA AGTCCCTTCC CTCTAATTCC CCATCCCTAG 2580
   ATACATCCTG GGAAGGACCC TACCCAGTCA TTTTATCTAC CCCAACTGCG GTTAAAGTGG 2640
   CTGGAGTGGA GTCTTGGATA CATCACACTT GAGTCAAATC CTGGATACTG CCAAAGGAAC 2700
   CTGAAAATCC AGGAGACAAC GCTAGCTATT CCTGTGAACC TCTAGAGGAT TTGCGCCTGC 2760
   TCTTCAAACA ACAACCAGGA GGAAAAATCG AAGCTGTAAA ACTACAAATG GAGCCCAAGA 2820
   TGCAGTCCAA GACTAAGATC TACCGCAGAC CCCTGGACCG GCCTGTTAGC CCACGATCTG 2880
   ATGTTAATGA CATCAAAGGC ACCCCTCCTG AGGAAATCTC AGCTGCACAA CCTCTACTAC 2940
   GCCCCAATTC AGCAGGAAGC AGTTAGAGCG GTCGTCGGCC AACCTCCCCA ACAGCACTTA 3000
   GGTTTTCCTG TTGAGATGGG GGACTGAGAG ACAGGACTAG CTGGATTTCC TAGGCTGATT 3060
   AAGAATCCCT AAGCCTAGCT GGGAAGGTGA CCACATCCAC CTTTAAACAC GGGGCTTGCA 3120
   ACTTAGCTCA CACCTGACCA ATCAGAGAGC TCACTAAAAT GCTAATTAGG CAAAGACAGG 3180
   AGGTAAAGAA ATAGCCAATC ATTTATTGCC TGAGAGCACA GCAGGAGGGA CAATGATCGG 3240
   GATATAAACC CAAGTTTTCG AGCCGGCAAC GGCAACCCCC TTTGGGTCCC CTCCCTTTGT 3300
   ATGGGAGCTC TGTTTTCATG CTATTTCACT CTATTAAATC TTGCAACTGC AAAAAAAAAA 3360
   AAAAAAAAAA AA 3372
(2) INFORMATIONS POUR LA SEQ ID NO: 10:
   (i) CARACTERISTIQUES DE LA SEQUENCE:
      (A) LONGUEUR: 2372 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 10:
   ACTGCACTCT TCTGGTCCAT GTTTCTTACG GCTCGAGCTG AGCTTTTGCT CACCGTCCAC   60
   CACTGCTGTT TGCCACCACC GCAGACCTGC CGCTGACTCC CATCCCTCTG GATCCTGCAG  120
   GGTGTCCGCT GTGCTCCTGA TCCAGCGAGG CGCCCATTGC CGCTCCCAAT TGGGCTAAAG  180
   GCTTGCCATT GTTCCTGCAC GGCTAAGTGC CTGGGTTTGT TCTAATTGAG CTGAACACTA  240
   ATCACTGGGT TCCATGGTTC TCTTCTGTGA CCCACGGCTT CTAATAGAAC TATAACACTT  300
   ACCACATGGC CCAAGATTCC ATTCCTTGGA ATCCGTGAGG CCAAGAACTC CAGGTCAGAG  360
   AATACGAGGC TTGCCACCAT CTTGGAAGCG GCCTGCTACC GTCTTGGAAG TGGTTCACCA  420
   CCATCTTGGG AGCTCTGTGA GCAAGGACCC CCCGGTAACA TTTTGGCAAC CAACGACGGA  480
   CATCCAAAGT GATGGGAAAC GTTCCCCGCA AGACAAAAAC GCCCCTAAGA CGTATTCTGG  540
   AGAATTGGGA CCAATTTGAC CCTCAGACAC TAAGAAAGAA ACGACTTATA TTCTTCTGCA  600
   GTGCCGCCTG GCACTCCTGA GGGAAGTATA AATTATAACA CCATCTTACA GCTAGACCTC  660
   TTTTGTAGAA AAGGCAAATG GAGTGAAGTG CCATAAGTAC AAACTTTCTT TTCATTAAGA 720
   GACAACTCAC AATTATGTAA AAAGTGTGAT TTATGCCCTA CAGGAAGCCT TCAGAGTCTA 780
   CCTCCCTATC CCAGCATCCC CGACTCCTTC CCCAACTAAT AAGGACCCCC CTTCAACCCA 840
   AATGGTCCAA AAGGAGATAG ACAAAAGGGT AAACAGTGAA CCAAAGAGTG CCAATATTCC 900
   CCAATTATGA CCCCTCCAAG CAGTGGGAGG AAGAGAATTC GGCCCAGCCA GAGTGCATGT 960
   GCCTTTTTCT CTCCCAGACT TAAAGCAAAT AAAAACAGAC TTAGGTAAAT TCTCAGATAA 1020
   CCCTGATGGC TATATTGATG TTTTACAAGG GTTAGGACAA TTCTTTGATC TGACATGGAG 1080
   AGATATAATG TCACTGCTAA ATCAGACACT AACCCCAAAT GAGAGAAGTG CCACCATAAC 1140
   TGCAGCCTGA GGGTTTGGCG TCTCTGGTAT CTCAGTCAGG TCAATGGATA NGGATGACAA 1200
   CAGAAGGAAA GANAATGATT CCCCACAGGC CAGCAGGCAG TTCCCAGTCT AGACCCTCAT 1260
   TGGGACACAG AATCAGAACA TGGAGATTGG TGCTGCAGAC ATTTGCTAAC TTGTGTGCTA 1320
   GAAGGACTAA GGAAAACTAG GAAGAAGTCT ATGAATTACT CAATGATGTC CACCATAACA 1380
   CAGGGAAGGG AAGAAAATCC TACTGCCTTT CTGGAGAGAC TAAGGGAGGC ATTGAGGAAG 1440
   CGTGCCTCTC TGTCACCTGA CTCTTCTGAA GGCCAACTAA TCTTAAAGCG TAAGTTTATC 1500
   ACTCAGTCAG CTGCAGACAT TAGAAAAAAC TTCAAAAGTC TGCCGTAGGC CCGGAGCAAA 1560
   ACTTAGAAAC CCTATTGAAC TTGGCAACCT CGGTTTTTTA TAATAGAGAT CAGGAGGAGC 1620
   AGGCGGAACA GGACAAACGG GATTAAAAAA AAGGCCACCG CTTTAGTCAT GACCCTCAGG 1680
   CAAGTGGACT TTGGAGGCTC TGGAAAAGGG AAAAGCTGGG CAAATTGAAT GCCTAATAGG 1740
   GCTTGCTTCC AGTGCGGTCT ACAAGGACAC TTTAAAAAAG ATTGTCCAAG TAGAAGTAAG 1800
   CCGCCCCTTC GTCCATGCCC CTTATTTCAA GGGAATCACT GGAAGGCCCA CTGCCCCAGG 1860
   GGACAAAGGT CTTTTGAGTC AGAAGCCACT AACCAGATGA TCCAGCAGCA GGACTGAGGG 1920
   TGCCTGGGGC AAGCGCCATC CCATGCCATC ACCCTCACAG AGCCCTGGGT ATGCTTGACC 1980
   ATTGAGGGCC AGGAAGGTTG TCTCCTGGAC ACTGGTGCGG TCTTCTTAGT CTTACTCTTC 2040
   TGTCCCGGAC AACTGTCCTC CAGATCTGTC ACTATTCTGA GGGGGTCCNT AAGACGGGCA 2100
   GTCACTAGAT ACTTTTTCCC AGCCACTAAG TTATGAACTG GGGAGCTTTA TTCTTTTCAC 2160
   ATGCTTTTCT AATTATGCTT GAAAGCCCCA CTACCTTGTT AGGGAGAGAC ATTCTAGCAA 2220
   AAGCAGGGGC CATTATACAC CTGAACATAG GAGAAGGAAC ACCCGTTTGT TGTNCCCCTG 2280
   CTTGAGGAAG GAATTAATCC TGAAGTCTGG GCAACAGAAG GACAATATGG ACGAGCCAAA 2340
   GAATGCCCGT CCTGTTCAAG TTAAACTAAA GG                               2372
(2) INFORMATIONS POUR LA SEQ ID NO: 11:
   (i) CARACTERISTIQUES DE LA SEQUENCE:
      (A) LONGUEUR: 7582 paires de bases
      (B) TYPE: nucléotide
      (C) NOMBRE DE BRINS: simple
      (D) CONFIGURATION: linéaire
   (ii) TYPE DE MOLECULE: ARNm (en ADN)
   (iii) HYPOTHETIQUE: NON
   (xi) DESCRIPTION DE LA SEQUENCE: SEQ ID NO: 11:
(2) INFORMATIONS POUR LA SEQ ID NO: 12:
   (i) CARACTERISTIQUES DE LA SEQUENCE:
      (A) LONGUEUR: 2563 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 12:
   ACTGCACTCT TCTGGTCCAT GTTTGTTACG GCTCGAGCTG AGCTTTTGCT CGCCATCCAC 60
   CACTGCTGTT TGCCACCGTT GCAGACCCAC TGCTGACTTC CATCCCTCTG GATCTGGCAG 120
   GGTGTCTGCT GTGCTCCTGA TCCAGCGAGG GGCCCATTGC CACTCCCAAT CGGGCTAAAG 180
   GCTTGCCATT GTTCCTGCAT GGCTAAGTGC CCAGGTTCAT CCTAATTGAG CTGAACACTA 240
   GTCACTGGGT TCCACAGTTC TCTTCCATGA ACCACGGCTT TTAATAGAGC TATAACACTC 300
   ATCGCAAGGC CCAAGATTCC ATTCCTTGGA ATCTGTGAGG CCAAGAACCC TAGGTCAGAG 360
   AACACGAGGC TTGCCACCAT CTTGGAAGCA GCCTGCCACC ATCTGGGAAG CGGCCTGCCA 420
   CCATCTTGGA AGCCGCCCGC CACCATCTTG GGAGCTCTGG GAGCAAGGAC CTCCCCGCAA 480
   CCCAGTAACA TTTAGCGACC ACGAAGGGAC CTCCAAAGCG GTAATATTGG ACCACTTTCA 540
   CTTGCTATTC TGTCCTATCC TTCCTTAGAA TTGGAGGAAA ATACCGGACA CCTGTCGGCC 600
   GGTTAAAAAC GATTAGCGTG GCCTCCGGAC TTAAGAATCA GGTGTGAGGC TATCTGGGGA 660
   AGGGCTTTCT AACAACCCCC AACCRTTCTG GGTTGGGAAT GTTGGTCTGC CTGGAGCCAG 720
   CTTCCACTTT CAATTTTCCT GGGGAAGCCA AGGGCCGACT AGAGGCAGAA AGCTGTTGTC 780
   CCAAATTCCC GGCAGTAGCC GGTTGAGATC ATGGCGCAGC CAGAAGTCTT TACTCCACAG 840
   TCACCCATGC ATGCGCCCCT ATCTTTCCTT CTGACCCATA CCTCCTGGGT CCTAACCATG 900
   ACTTTCTTAA AAGGGTAGCC CCAAAATTCT CCTTACCTCT GAATCTACTT CCTCTGATCC 960
   CTGCCTCCTA GGTGCTAATG GTTCAGACTT TCATTTCCTC TAGCAAGTTG TATYTCCAAA 1020
   GGGATATAAG GAAGCTCTAC ACTGTATCCT TAGGCATCTA GGCTCTAAAC CCAGGGAGTC 1080
   TTGTCCCTGA TGTCCCAACC GATTTAGGTA TATAGTTCTC GACATGGGCA GTTATGTGGG 1140
   ACCCATTCCC CACCACCCTT GCCAGGGCCC CAAGTTTGTA AATGGCTAAG AGAGGAAAGT 1200
   GAGAGAGAGA GAGACAGAGT GAGACACAGA GAGAGGGAGA GACAGAGAGA GAGACAGAGA 1260
   GGAGAGAGAC ACAGAGAGGG GAGAGACACA GAGAGGAGAA GGGGGCAGAG AGACCAAGAG 1320
   GGAGTCYMAG AGAGAGAGAA AGAAGAAGAA ATAGTAGAAA AAAAAGTGTG CCCTATTCCT 1380
   TTAAAAGCCA GGGTAAATTT AAAAAACCTA TACTTGATAA TTGAAGGTCT TCTCCATGAC 1440
   CCTGTAACAC TCTAATACTA CCTTGTTCTC AGTGTAAACA AGGGTGTTAG CCTGAAAACA 1500
   CTGAGACCGC TGACACCCAT AGCTTTCCTA TAAAAAATCC TTAACCCAGT AACCCGCAGA 1560
   TGGCCCGCAT GCATTCAATC TGTAGTGGCA ACTGCTTTGC TAACAAGAAT AAAGTGGAAA 1620
   AGTAACTTTT AGAGGAAACC TCATTGTGAG CACACCTCAC CAGTTCAGAA TTATTCTAAG 1680
   TCAAAAAAGC AAAAAGGTAG CTTACTAACT CAAAAATCTT AAAGTATGGG GTTATTTTGT 1740
   TAGAAAAAGG TAATTTAACA CTAATCACTG ATAATTCCCT TAACCCAGAA GATTTCCTAA 1800
   CAGGAGATTT AAATCTTAAT TACCATACAA AGGTCTGACC AGACCTAGGA GGAACTCCCT 1860
   TCAGTACAGG ATGATAGATG GTTCCTCCCA GGTGAATGAA AAAAAAATCA CAATGGGTAT 1920
   TCAGTAATTG ATAGGGAGAC TCTTGTGGAA GCAGAGTTAG AAAAACTGCC TAATAATTGG 1980
   TCTCCCCAAA CCTGCGAGCT GTTTGCACTC AGCCAAGCCT TAAAGTACTT CTAGAATCAA 2040
   AAAGATTATC TCAATCCTGA CTCAAAAGGT TACCTACACC CTCTGTGAAA CGAATTTACT 2100
   TAAGAACTGT TTATGGGACT GCATCTTGAT GGGGCAGCTG GGTTGTCATG AAATACTCAG 2160
   GAATGCAGCC TAGCTCTAGG ACTCACCCCT GAGCACAAAG GCAATGTTGG GCATGCTGGT 2220
   AAAGGACCAC TAGAATCCAG CAGTCCGAAC CCTTTCTTTG GGTTAAGAAA GGCGGGAAAA 2280
   CAGGCGCAGG ACTGCTACAT TGGTAAGCGT AACTAATCCA ATAAGCAGAG GTCCATGGGT 2340
   GGTGACACAC TCTGGAAAGG AATAAGCATT AGRACCATAG AGGACGCTCT ACGACTAATG 2400
   CTCGTCGGAA AATGACTAGA GGTGCTGGCA TCCCTATGTT CTTTTTTCAG ATGGGAAATG 2460
   TTCCCCCTCA AGGCAAAAAC ACCCCTAAGA TGTATTCTGG ACAATTGGGA CCAATTTGAC 2520
   CCTCAGACTC TAAGAAAGAA ACGACTTATA TTCTTCTGCA GTG 2563
(2) INFORMATIONS POUR LA SEQ ID NO: 13:
   (i) CARACTERISTIQUES DE LA SEQUENCE:
      (A) LONGUEUR: 2585 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 13:
   TCAGGGATAG CCCCCATCTA TTTGGCCAGG TATTAGCCCA AGACTTGAGC CAGTTCTCAT 60
   ACTTGGACAC TCTTGTCCTT TGGTATGTGG ATGATCTACT TTTAGCCACC TGTTCAGAAA 120
   CCTTGTGCCA TCAAGCCAAC CAAGTGCTCT TAAACTTCCT CGCCACCTGT GGCTACAAGG 180
   TTTCCAAACC AGAGGCTCAG CTCTGCTTAC AGCAGGTTAA ATACTTAGGG CTAAAATTAT 240
   CCAAAGGCAC CAGGGCCCTC AGTGAGGAAC GTATCCAGCC TATACTGGCT TATCCTCATC 300
   CCAAAACCCT GAAGCAATTA AGAGGGTTCC TTGGCATAAA AGGCTGCTGT TGAATATGGA 360
   TTCCCAGGTA CAATGAAATA GCCAGGCCAT TATACACACT AATTACGGGA ACTCAGAAAG 420
   CCAATACCCA TTTAGTAGAA TGGACACCTG AAGCAGAAGC GGCTTTCCAG GCCCTAAAGA 480
   AGGCCCTAAT CCAAGCCCCA GTGTTAAGCT TGCCAATGGA GCAAGACTTT TCTTTATATG 540
   TCACAGAAAA AAAAACAGGA ATAGCTCTAG AAGTCCTTAC ACAGGTCCGA GGGACCAGCT 600
   TACAACACAT GGCATACCTG AGTAAGGAAA CTGATGTAGT GGCAAAGGGT TGGACTCATT 660
   GTTTACAGGT AGTGGCAGCA GTAGCAGTCT TAGCATCTGA AGCAGTTAAA ATGATACAGG 720
   GAAGANATCT TACTGTGTGG ACATCTCATG ATGTGAACGG CATACTCACT GCTAAAGGAG 780
   ACTGTGGCTG TCAGACAACC ATTTGCTTAA ATATCAGGCT CTATCACTTG AANGGCCAGT 840
   GCTGCCACTG TGCACTTGTG CAACTCTTAA CCCACCCACA TTTCTTCCAG ACAATGAAGA 900
   AAAGATAGAA CATAACTGTC AACAAGTGAT TGTTCAAACC TACACCGCTC GAAGGGACCT 960
   TCTAGAGGTT CCCTTGACTG ATCCTGAGCT CAACTTCTAT ACTGATGGAA GTTCCTTTTG 1020
   TAGAAAAAGG ACTTCGAAAG GCGGGTATGC AGTGGCCAGT GATAATGGAA TACTTGAAAG 1080
   TAATCCCTTC ACTCCAGAAA CTAGCATTCA GCTGGCAGAA TTAATAGCCT TCACTTGGGC 1140
   ATTAGAACAC AGGAGAAGGA AAAGGAGTAA ATATATATAC AGACTCCAAG TATGCTTACT 1200
   TAGTCCTCCA TGCCCATGCA GCAATATAGA GAGAAAGCGA ATTCCTAACT TCTGAGGGAA 1260
   CACCTATCAA ACATCAGGAA GCCATTAGGA GATTATTACT GGCTGTACAG AAACCTAGAG 1320
   GTGGCAGTCT TACATGGCCG AGATCATCAG AAAGGAAAAG AAAGGGAAAT AGAAGGGAAC 1380
   TGCCAAGTGG ATATTGAAGC CAAAAGAGCT GCAAGGCGGG ACCCTCCATT AGAAATGCTT 1440
   ATAGAAGGAC CCCTAGTACA GGGCAATCCC CTTCAGGAAA CCAAGCCCCA ATACTCAGCA 1500
   GAAGAAATGG AATGGGGAAC CTCATGAGGA CATAGTTTCC TCCCCTCAGG ATGGCTAGCC 1560
   ACCAAAGAAG GAAAAATACT TTTGCCTGCA GCTAACCAAT GGAAATTACT TAAAACCCTT 1620
   CACCAAACCT TTCGCTTAGG CATTGATAGC ACCCATCAGA TGGCTAAATC ATTATTTACT 1680
   AGACCACACC TTTTCAAAAC TATCAAGCAG ACAGTTAGGG CCTGTGAAGT GTGCCAAAGA 1740
   AATAATCCCC TGCCTTATCG CCAAACTCCT TCAGGAGAAA AAAGAACAGG CCATTACCCA 1800
   GGAGAAGAGT GGCAACTAGA TTTTACCCAC ATGCCCAAAT CTCAGGGATT TCAGTATCTA 1860
   CTAGTCTGGG TAGATACTTT CACTGGTTGG GCGGAGGCCT TCCCTTGTAG GACAGAACAG 1920
   GCCCATGAGG TAATAAAGGC ACTAATTCAT GAAATAATTC CCAGATTTGG ATTTCCCCAA 1980
   GGCTTACAGA GTGATAACGG CCCCACTTTC AAGGCTACAG TAACCCAGGG AGTATCCCAG 2040
   ACATTAGACA TACAATATCA CTTACACTGA GCCCGGAGGC CACAATCCTC AGGAAAGTTG 2100
   AGAAAATGAA TGAAACGCTC AAATGACATC TAAAAAAGCT AACCTAAGAA ACCCACCTCT 2160
   CATGGTTTGC TCTGTTGCCT ATAGCCTTAG TAAGAATCCG AAACTCTCCC CAAAAAGCGG 2220
   GACTCAGCCC ATACGAAATG CTGTATGGAC GGCCCTTCCT AACCAATGAC CTTGTGCTTG 2280
   ACCTAGAGAT GGCCAACTTA GTTGCAGATA TCCCTCCTTA GCCAAATATC AACAAGTTCT 2340
   TAAAACGTCA CAGGGAACCT GTCCCTGAGA GGAGGGAAAG GAATTATTCC AACCTGGTGA 2400
   CATGGTATTA GTGAAGTCCC TTCCCTCCAA CTCCCCATCC CCTGGATACA TCCTGGGAAG 2460
   GACCCTACTC AGTCATTTTA TCTATCCCAA CCGCGGTTAA AATGGCTGGA GTAGAATCTT 2520
   GGATACATCA CATTCGAGTC AAACCCTAGA TACTGCCACA AGGAACCTGA AAATCCAGGA 2580
   GACAA 2585
(2) INFORMATIONS POUR LA SEQ ID NO: 14:
   (i) CARACTERISTIQUES DE LA SEQUENCE:
      (A) LONGUEUR: 2575 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 14:
   GGGATAGCCC CCATCTATTT GGCCAGGCAT TAGCCCAAGA CTTGAAGCCA ATTCTCATAC 60
   CTGGACACTC TTCTCCTTTG GTATGTGGAT GATTTACTTT TAGCTTCCTG TTCAGAAACC 120
   TTGTGCCATC AAGCCACCCA AGCACTCTTA AATTTCCTCG CTACCTGTGG CTACAAGGTT 180
   TCCAAACCAA AGACCCAGCT CTGCTCACAG CAGGTTAAAT ACTTGGGGCT AAAATTATCC 240
   AAAGGCACCA GGGCCCTCAG TGAGGAACGT ATCAAGCCTA TACTGGCTTA TCCTCATCCC 300
   CAAATCCTAA AGCAACTAAG AGAGTTCCTT AGCATAACAG GTTTCTGCTG AATATGGATT 360
   CCCAGGTATG GCAAAATAGC CAGACCATTA TATACGCTAA TTAAGGAAAC TCAGAAAGCC 420
   AATACCCATT TAGTAAGATG GATACCTGAA GCAGAAGCAG CTTTCCAGGC CCTAAAGAGG 480
   GCCCTAACCC AAGCCCCAGT GTTAAGCTTG CCAACAGGGC AAGACTTTAC TTCGTATGTC 540
   ACAGAAAAAA CAGGAAATAG CTCTAGGAGT CCTTACACAA GTCTGAGGGA TGAGCTTGCA 600
   ACCCATGGCA TACCTGAGTA AGGAAATTGA TGTAGTGGCA AAGGGTTGGC CTCATTGTTT 660
   ATGGGTAGTG GCGGCAGTAG CAGTCTTAGC ATCTGAAGCA GTTAAAATGA TACAGGGAAG 720
   AGATCTTACT GTGTGGACAT CTCATGATGT GAATGGCATA CTCACTGCTA AAGGAGACTT 780
   GTGGCTGTCA GACAACCATT TACTTAAATA TCAGGCTGTA TTACTTGAAG GGCCAGTGCA 840
   GCAACTGCGC AGTTGTGCAG CTCTTAACCC AGCCACATTT CTTCCAGACA ATGAAGATAG 900
   AACATAACTG CCAACAAGTA ATTTCTCAAA CCTAGGCCGC TCGAGGGAAC CTTTTAGAGG 960
   TTCCCTTAAC TGATCCCGAC CTCAACTTGT ATACTGATGG AAGTTCCTTT GTAGAAAAAG 1020
   GACTTTGAAA AGTGGGGTAT GCAGTGCTCA GTGATAATGG AATACTTGAA AATAATCCCT 1080
   TCATTCCAGG AACCAGCGTT CAGCTGGCAG AATTAATAGC CCTCACTCGG GCATTAGAAT 1140
   TAGGAGAAGG AAAAAGGGTA AATACACATA CAGATTCTAA GTATGTTTAC TTAGTCCTCC 1200
   GTGCCCACGC AGCAATATGG AGAGAAAGGG AATGCTTAAC TTCTGAGGGA ACACCTATCA 1260
   AACATCAGGA AGTTATTAGG AGATTATTAT TGGCTATACA GAAACCTAAA GAGGTGGCAG 1320
   TCTTACACTG CTGGGGTGGT CAGAAAGAAA AGGAAAGGGA AATAAAAGGG AACTGCCAAG 1380
   CGGATATTGA AGCCAAAAGA GCCGCAAGGC AGGACCCTCC ATTAGAAATG CTTATAGAAG 1440
   GACCCCTAGT ATGGGGTAAT CCCCTCCGGG AAACCAAGCC CCAATACTTA GAAAAAGAAA 1500
   TAGAATGGGG AACCTCACGA GGACATAGTT TCCTCCCCTC AGGATGGCTA GCCACCGAAG 1560
   AAGGAAAAAT ACTTTTGCCT GCAGCTAACC AATGGAAATT ACTTAAAACC CTTCACCAAA 1620
   CCTTTCACTT AGACATTGAT AGCACCCATC AGATGGCCAA ATCATTATTT ACTGGACCAG 1680
   GCCTTTTCAA AACTATCAAG CAGCTAGTCA GGGCCTGTGA AGTGTGCCGA AGAAATAATC 1740
   CCATGCCTTA TCACCAAGCT CCTTCAGGAG AACAAAGAAC AGGCCATTAC CCAGGAGAAG 1800
   RVTGGCAACT AGATTTTACC CACATGCCCA AATCTCAGGG ATTTCAGTAT CTACTAGTTT 1860
   GGGTAGATAC TTTCACTGGT TGGGCAGAGA CCTTCCCCTG TAAGACAGAA AAGTCCCAAG 1920
   AGGTAATAAA GGCATTAGTT CATGAAATAA TTCCCAGATT CAGACTTCCC TGAGGCTTAC 1980
   AGAGTGACAA TGGCCCTGCT TTCAAGGCTA CAGTAACCCA GGAGTATCCC AGGTGTTAGG 2040
   TATACAATAT CACTTACACT GCGCCTGGAG GCAGTCCTCA GGGAAGGCCG AGAAACTGAA 2100
   TGAAACACTC AAACGACATC TAAAAAAAGC TAACCCAGGA AAACCACCTC ACATGGCCTG 2160
   CTCTGTTGCC TATAGCCTTA CTAAGAATCC AAAACTCTCC CCAAAAAGCA GGACTTAGCC 2220
   CATACGAAAT GCTATATGGA TAGCCCTTCC TAACCAATGA CCTTGTGCTT GACTGAGAGA 2280
   GAGCCAACTT AGTTGCAGAC ATCACCTCCT TATCCAAATA TCAACAAGTT CTTAAAACAT 2340
   TACAAGGAGC CTGTCCCCGA GAAGAGGGGA AGGAACTATT CCACCCTGGT GACATGGTAT 2400
   TAGTCAAGTC CCTTCCCTCT AATTCTCATT GCCTAGATAT ATCCTGGGAA GGACCCTACC 2460
   CAGTCATTTT ATCTACCCCA ACCGCAGTAA AAGTGGCTGG AGTGGAGTCT TGGATACATC 2520
   ACACTCGAGT CAAACCCTGG ATATTACCAA AGGAACCTGA AAATCCAGGA GACAA 2575
(2) INFORMATIONS POUR LA SEQ ID NO: 15:
   (i) CARACTERISTIQUES DE LA SEQUENCE:
      (A) LONGUEUR: 783 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 15:
   TGAGAGACAG GACTAGCTGG ATTTCCTAGG CYGACTAAGA ATCCYTAAGC CTAGSTGGGA 60
   AGGTGACCAC RTCCACCTTT AAACACGGGG CTTGCAACTT AGYTCACACC TGACCAATCA 120
   GAGAGCTCAC TAAAATGCTA ATTAGGCAAA GACAGGAGGT AAAGAAATAG CCAATCATYT 180
   ATTGCMTGAG AGCACAGCAG GAGGGACAAY RATCGGGATA TAAACCCARG YHTTCGAGCY 240
   GGCAACRGCA GMCCCCCTTT GGGTCCCYTC CCTTTGTATG GGAGCTCTGT TTTCATGCTA 300
   TTTCACTCTA TTAAATCTTG CARCTGCRCT CTTCTGGTCC ATGTTTCTTA CGGCTYGAGC 360
   TGAGCTTTYG CTCRCCRTCC ACCACTGCTG TTTGCCRCCA CCGCANACCY GCCGCTGACT 420
   CCCATCCCTC TGGATCMTGC AGGGTGTCCG CTGTGCTCCT GATCCAGCGA RGCRCCCATT 480
   GCCGCTCCCA ATYGGGCTAA AGGCTTGCCA TTGTNCCTGC AYGGCTAAGT GCCTGGGTTY 540
   RTYCTAATTG AGCTGAACAC TANTCACTGG GTTCCATGGT TCTCTTCTGT GACCCACRGC 600
   TTCTAATAGA RCTATAACAC TYACCRCATG GCCCAAGRTT CCATTCCTTG GAATCCRTRA 660
   RGSCAACGAA CYCCASGTCA GAGAAYACGA RGCTTGCCAC CATCTTGGAA GCGGCCTGCT 720
   ACCATCTTGG AAGTGGTTCA CCACCATCTT GGGAGCTCTG TGAGCAAGGA CCCCCMRGTR 780
   ACA 783
(2) INFORMATIONS POUR LA SEQ ID NO: 16:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 16:
      TGTCCGCTGT GCTCCTGATC 20
(2) INFORMATIONS POUR LA SEQ ID NO: 17:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 17:
      ATGCACTCTG GCTGGGCCAA T 21
(2) INFORMATIONS POUR LA SEQ ID NO: 18:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 18:
      ACCATTTGAC CCTCAGACAC T 21
(2) INFORMATIONS POUR LA SEQ ID NO: 19:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 19:
      AACCCTTTGC CACTACATCA ATTT 24
(2) INFORMATIONS POUR LA SEQ ID NO: 20:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 20:
      TCAGGGATAG CCCCCATCTA T 21
(2) INFORMATIONS POUR LA SEQ ID NO: 21:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 21:
      TTGTCTCCTG GATTTTCAGG TT 22
(2) INFORMATIONS POUR LA SEQ ID NO: 22:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 22:
      GGACCCTACC CAGTCATTTT 20
(2) INFORMATIONS POUR LA SEQ ID NO: 23:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 23:
      ATCAGGAGCA CAGCGGACAC 20
(2) INFORMATIONS POUR LA SEQ ID NO: 24:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 24:
      GGACATCCAA AGTGATACAT CC 22
(2) INFORMATIONS POUR LA SEQ ID NO: 25:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 25:
      AATGTATGGC CTGAAGTGCA G 21
(2) INFORMATIONS POUR LA SEQ ID NO: 26:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 26:
      CTTCCCAGGA TGTATCACTT TG 22
(2) INFORMATIONS POUR LA SEQ ID NO: 27:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 27:
      CACTGCAGAA GAATATAAGT CGTT 24
(2) INFORMATIONS POUR LA SEQ ID NO: 28:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 28:
      GCTTCCAAGA TGGTGGCAAG C 21
(2) INFORMATIONS POUR LA SEQ ID NO: 29:
   (i) CARACTERISTIQUES DE LA SEQUENCE:
      (A) LONGUEUR: 678 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 29:
   TCAGGGATAG CCCCCATCTA TTTGGCCAGG CATTAGCCCA AGACTTGAGC CAGTTCTCAT   60
   ACCTGGATAT TCTTGTCCTT TGGTATGCGG ATGATTTACT TTTAGCCGCC CGTTCAGAAA  120
   CCTTGTGCCA TCAAGCCACC CAAGTGCTCT TAAATTTCCT CGCCACCTGT GGCTACAAGG  180
   TTTCCAAACC AAAGGCTCAG CTCTGCTCAC AGCAGAAGGC TATTTACCCT AAATACTTAG  240
   GGCTGAAATT ATCCAAAGGC ACCAGGGCCC TCAGTGAGGA ATGTATCCAG CCTATACTGG  300
   CTTATCCTTA TCCCAAAACC CTAAAACAAC TAAGAAGGTT CCTTGGCATA ATAGGCATAA  360
   CAGGCATAAC AGGTTTCTGC TGAATATGGA TTCCCAAGTA CGGCAAAATA GCCAGACCAT  420
   TATATACACT AATTAAGGAA ACTCAGAAAG CCAATACCCA TTTAGTAAGA TGGACACCTG  480
   AAGCAGAGGC AGCTTTCCAG GCCGTAAAGA ACACCCTAAC CCAAGCCCCA GTGTTAAGCT  540
   TGCCAGCGGG GCAAGACTTT TCTTTCTGTG TCACAGAAAA AATAGGAATA GCTNTAGGAG  600
   TCCTTACACA GGTCCGAGGG ACCAGCTTGC AACCCATGGC ATACCTGAGT AAGGAAATTG  660
   ATGTAGTGGC AAAGGGTT                                                678
(2) INFORMATIONS POUR LA SEQ ID NO: 30:
   (i) CARACTERISTIQUES DE LA SEQUENCE:
      (A) LONGUEUR: 536 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 30:
   CCAATCTCCA TGTTGTATCC CCTTCCCCAA CTAATAAGGA CCCCCCTTTC AACCCAAACA    60
   GTCCAAAAGG ACATAGACAA AGGAGTAAAC AATGAACCAA AGAGTGCCAA TATTCCCTGG   120
   TTATGCACCC TCCAAGCGGT GGGAGAAGAA TTCGGCCCAG CCAGAGTGCA TGTACCTTTT   180
   TCTCTCTCAC ACTTGAAGCA AATTAAAATA GACCTAGGTA AATTCTCAGA TAGCCCTGAT   240
   GGCTATATTG ATGTTTTACA AGGATTAGGA CAATCCTTTG ATCTGACATG GAGAGATATA   300
   ATATTACTGC TAAATCAGAC GCTAACCTCA AATGAGAGAA GTGCTGCCAT AACTGGAGCC   360
   CGAGAGTTTG GCAATCTCTG GTATCTCAGT CAGGTCAATG ATAGGATGAC AACGGAGGAA   420
   AGAGAACGAT TCCCCACAGG GCAGCAGGCA GTTCCCAGTG TAGCTCCTCA TTGGGACACA   480
   GAATCAGAAC ATGGAGATTG GTGCCGCAGA CATTTAAAGC TTTCCCCGGG TACCGA       536
(2) INFORMATIONS POUR LA SEQ ID NO: 31:
   (i) CARACTERISTIQUES DE LA SEQUENCE:
      (A) LONGUEUR: 591 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 31:
   CCATGGCCAT CTACACTGAA CAAGATTTAT ACAATCATGT CGTACCTAAG CCCCACAACA   60
   AAAGAGTACC CATTCTTCCT TTTGTTATCA GAGCAGGAGT GCTAGGCAGA CTAGGTACTG  120
   GCATTGGCAG TATCACAACC TCTACTCAGT TCTACTACAA ACTATCTCAA GAAATAAATG  180
   GTGACATGGA ACAGGTCACT GACTCCCTGG TCACCTTGCA AGATCAACTT AACTCCCTAG  240
   CAGCAGTAGT CCTTCAAAAT CGAAGAGCTT TAGACTTGCT AACCGCCAAA AGAGGGGGAA  300
   CCTGTTTATT TTTAGGAGAA GAACGCTGTT ATTATGTTAA TCAATCCAGA ATTGTCACTG  360
   AGAAAGTTAA AGAAATTCGA GATCGAATAC AATGTAGAGC AGAGGAGCTT CAAAACACCG  420
   AACGCTGGGG CCTCCTCAGC CAATGGATGC CCTGGGTTCT CCCCTTCTTA GGACCTCTAG  480
   CAGCTCTAAT ATTGTTACTC CTCTTTGGAC CCTGTATCTT TAACCTCCTT GTTAAGTTTG  540
   TCTCTTCCAG AATTGAAGCT GTAAAGCTAC AGATGGTCTT ACAAATCTAG A           591
(2) INFORMATIONS POUR LA SEQ ID NO: 32:
   (i) CARACTERISTIQUES DE LA SEQUENCE:
      (A) LONGUEUR: 364 paires de bases
      (B) TYPE: nucléotide
      (C) NOMBRE DE BRINS: simple
      (D) CONFIGURATION: linéaire
   (ii) TYPE DE MOLECULE: ADN
   (iii) HYPOTHETIQUE: NON
   (xi) DESCRIPTION DE LA SEQUENCE: SEQ ID NO: 32:
   CTAACCTGAG GATCCAGCAG CAGGACTGAG GGTGCCCGGG GCAAGTGCCA GCCCATGCCA 60
   TCACCCTCAG AGCCCCGGGT ATGTTTGACC ATTGAGAGCC AGGAAGTTAA CTGTCTCCTG 120
   GACACTGGCG CAGCCTTCTC AGTCTTACTT TCCTGTCCCA GACAATTGTC CTCCAGATCT 180
   GTCACTATCC GAGGGGTCCT AGGACAGCCA GTCACTACAT ACTTCTCTCA GCCACTAAGT 240
   TGTGACTGGG GAACTTTACT CTTTTCACAT GCTTTTCTAA TTATGCCTGA AAGCCCCACT 300
   CCCTTGTTAG GGAGAGACAT TTTAGCAAAA GCAGGGGCCA TTATACACCT GAACAAGCTT 360
   GAAA 364
(2) INFORMATIONS POUR LA SEQ ID NO: 33:
   (i) CARACTERISTIQUES DE LA SEQUENCE:
      (A) LONGUEUR: 538 acides aminés
      (B) TYPE: acide aminé
      (C) NOMBRE DE BRINS: simple
      (D) CONFIGURATION: linéaire
   (ii) TYPE DE MOLECULE: protéine
   (iii) HYPOTHETIQUE: NON
   (xi) DESCRIPTION DE LA SEQUENCE: SEQ ID NO: 33:
   Met Gly Leu Pro Tyr His Ile Phe Leu Cys Ser Val Leu Ser Pro Cys
   1 5 10 15
   Phe Thr Leu Thr Ala Pro Pro Pro Cys Arg Cys Met Thr Ser Ser Ser
   20 25 30
   Pro His Pro Glu Phe Leu Trp Arg Met Gln Arg Pro Gly Asn Ile Asp
   35 40 45
   Ala Pro Ser Tyr Arg Ser Leu Ser Lys Gly Thr Pro Thr Phe Thr Ala
   50 55 60
   His Thr His Met Pro Arg Asn Cys Tyr His Ser Ala Thr Leu Cys Met
   65 70 75 80
   His Ala Asn Thr His Tyr Trp Thr Gly Lys Met Ile Asn Pro Ser Cys
   85 90 95
   Pro Gly Gly Leu Gly Val Thr Val Cys Trp Thr Tyr Phe Thr Gln Thr
   100 105 110
   Gly Met Ser Asp Gly Gly Gly Val Gln Asp Gln Ala Arg Glu Lys His
   115 120 125
   Val Lys Glu Val Ile Ser Gln Leu Thr Gly Val His Gly Thr Ser Ser
   130 135 140
   Pro Tyr Lys Gly Leu Asp Leu Ser Lys Leu His Glu Thr Leu Arg Thr
   145 150 155 160
   His Thr Arg Leu Val Ser Leu Phe Asn Thr Thr Leu Thr Gly Leu His
   165 170 175
   Glu Val Ser Ala Gln Asn Pro Thr Asn Cys Trp Ile Cys Leu Pro Leu
   180 185 190
   Asn Phe Arg Pro Tyr Val Ser Ile Pro Val Pro Glu Gln Trp Asn Asn
   195 200 205
   Phe Ser Thr Glu Ile Asn Thr Thr Ser Val Leu Val Gly Pro Leu Val
   210 215 220
   Ser Asn Val Glu Ile Thr His Thr Ser Asn Leu Thr Cys Val Lys Phe
   225 230 235 240
   Ser Asn Thr Thr Tyr Thr Thr Asn Ser Gln Cys Ile Arg Trp Val Thr
   245 250 255
   Pro Pro Thr Gln Ile Val Cys Leu Pro Ser Gly Ile Phe Phe Val Cys
   260 265 270
   Gly Thr Ser Ala Tyr Arg Cys Leu Asn Gly Ser Ser Glu Ser Met Cys
   275 280 285
   Phe Leu Ser Phe Leu Val Pro Pro Met Thr Ile Tyr Thr Glu Gln Asp
   290 295 300
   Leu Tyr Ser Tyr Val Ile Ser Lys Pro Arg Asn Lys Arg Val Pro Ile
   305 310 315 320
   Leu Pro Phe Val Ile Gly Ala Gly Val Leu Gly Ala Leu Gly Thr Gly
   325 330 335
   Ile Gly Gly Ile Thr Thr Ser Thr Gln Phe Tyr Tyr Lys Leu Ser Gln
   340 345 350
   Glu Leu Asn Gly Asp Met Glu Arg Val Ala Asp Ser Leu Val Thr Leu
           355                 360                 365
   Gln Asp Gln Leu Asn Ser Leu Ala Ala Val Val Leu Arg Asn Arg Arg
       370                 375                 380
   Ala Leu Asp Leu Leu Thr Ala Glu Arg Gly Gly Thr Cys Leu Phe Leu
   385                 390                 395                 400
   Gly Glu Glu Cys Cys Tyr Tyr Val Asn Gln Ser Gly Ile Val Thr Glu
                   405                 410                 415
   Lys Val Glu Glu Ile Pro Asp Arg Ile Gln Arg Ile Ala Glu Glu Leu
               420                 425                 430
   Arg Asn Thr Gly Pro Trp Gly Leu Leu Ser Arg Trp Met Pro Trp Ile
           435                 440                 445
   Leu Pro Phe Leu Gly Pro Leu Ala Ala Ile Ile Leu Leu Leu Leu Phe
       450                 455                 460
   Gly Pro Cys Ile Phe Asp Leu Leu Val Asn Phe Val Ser Ser Arg Ile
   465                 470                 475                 480
   Glu Ala Val Lys Leu Gln Met Glu Pro Lys Met Gln Ser Lys Thr Lys
                   485                 490                 495
   Ile Tyr Arg Arg Pro Leu Asp Arg Pro Ala Ser Pro Arg Ser Asp Val
               500                 505                 510
   Asn Asp Ile Lys Gly Thr Pro Pro Glu Glu Ile Ser Ala Ala Gln Pro
           515                 520                 525
   Leu Leu Arg Pro Asn Ser Ala Gly Ser Ser
       530                 535
(2) INFORMATIONS POUR LA SEQ ID NO: 34:
   (i) CARACTERISTIQUES DE LA SEQUENCE:
      (A) LONGUEUR: 52 acides aminés
      (B) TYPE: acide aminé
      (C) NOMBRE DE BRINS: simple
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 34:
   Met Glu Pro Lys Met Gln Ser Lys Thr Lys Ile Tyr Arg Arg Pro Leu
   1               5                   10                  15
   Asp Arg Pro Ala Ser Pro Arg Ser Asp Val Asn Asp Ile Lys Gly Thr
               20                  25                  30
   Pro Pro Glu Glu Ile Ser Ala Ala Gln Pro Leu Leu Arg Pro Asn Ser
           35                  40                  45
   Ala Gly Ser Ser
       50
(2) INFORMATIONS POUR LA SEQ ID NO: 35:
   (i) CARACTERISTIQUES DE LA SEQUENCE:
      (A) LONGUEUR: 48 acides aminés
      (B) TYPE: acide aminé
      (C) NOMBRE DE BRINS: simple
      (D) CONFIGURATION: linéaire
   (ii) TYPE DE MOLECULE: peptide
   (iii) HYPOTHETIQUE: NON
   (xi) DESCRIPTION DE LA SEQUENCE: SEQ ID NO: 35:
   Met Leu Met Thr Ser Lys Ala Pro Leu Leu Arg Lys Ser Gln Leu His
   1 5 10 15
   Asn Leu Tyr Tyr Ala Pro Ile Gln Gln Glu Ala Val Arg Ala Val Val
   20 25 30
   Gly Gln Pro Pro Gln Gln His Leu Gly Phe Pro Val Glu Met Gly Asp
   35 40 45

## Description

La présente invention concerne un nouveau matériel nucléique, de type génomique rétroviral endogène, différents fragments nucléotidiques qui le comprennent ou qui sont obtenus à partir dudit matériel, ainsi que leur utilisation pour marquer la sclérose en plaques.

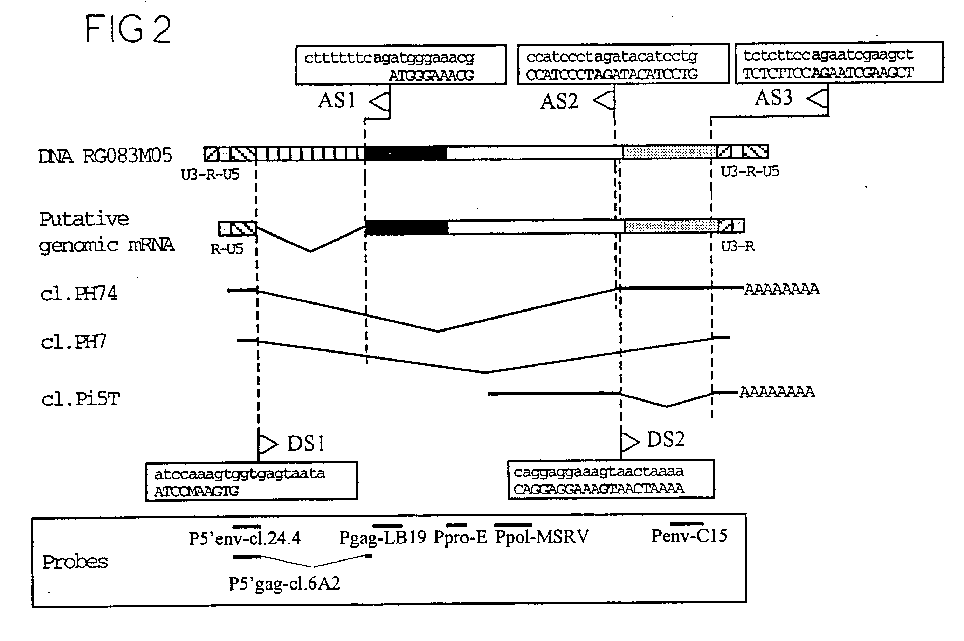

Le criblage de la banque d'ADNc à l'aide de la sonde Ppol-MSRV (SEQ ID NO: 29) a permis de détecter des clones chevauchant permettant la reconstruction d'un ARN génomique putatif de 7582 nucléotides. - Par séquence reconstruite, on entend la séquence déduite de l'alignement des clones chevauchants Cet ARN génomique présente la structure R-U5-gag-pol-env-U3-R. Une interrogation "blastn" sur plusieurs bases de données, à l'aide du génome reconstruit, montre qu'il existe une quantité importante de séquences génomiques (ADN) apparentées dans le génome humain. Environ 400 séquences ont été identifiées dans GenBank (cf figure 2) et plus de 200 séquences dans la banque EST (Expressed Sequence Tag), la plupart en antisens. Ces séquences sont trouvées sur plusieurs chromosomes, notamment les chromosomes 5, 7, 14, 16, 21, 22, X, avec une forte concentration apparente de LTR sur le chromosome X.

La séquence reconstruite (ARNm) est contenue intégralement à l'intérieur du clone génomique RGOB3M05 (gb AC00064) (9,6 kb), et présente une similitude de 96% avec deux régions discontinues de ce clone qui contient également des régions répétées à chaque extrémité. L'alignement des séquences expérimentales correspondant aux régions 5' et 3' de l'ARN génomique reconstruit avec l'ADN du clone RG083M05 a permis de déduire une séquence LTR et d'identifier des éléments caractéristiques des rétrovirus notamment ceux impliqués dans la transcription inverse, à savoir le PBS (Primer Binding Site) en aval du LTR 5' et le PPT (PolyPurine Tract) en amont du LTR 3'. On observe que l'élément U3 est extrêmement court en comparaison de celui observé chez les rétrovirus de type C des mammifères, et comparable en taille à la région U3 généralement décrite chez les rétrovirus de type D et les rétrovirus aviaires. La région PBS est homologue au PBS des rétrovirus aviaires, suggérant l'utilisation du tRNA^{Trp} comme amorce pour la transcription inverse. Par conséquent, cette nouvelle famille de HERV est nommée HERV-W (Human Endogenous RetroVirus).

L'analyse phylogénique dans la région pol a montré que la famille HERV-W est phylogéniquement liée aux familles ERV-9 et RTVL-H, et appartient donc à la famille des rétrovirus endogènes de type I. L'analyse phylogénétique de la trame de lecture ouverte (ORF) de env montre qu'elle est plus proche des rétrovirus simiens de type D et des rétrovirus de la réticuloendothéliose aviaire que des rétrovirus mammifères de type C, suggérant une structure de génome chimérique C/D.

Les arbres phylogéniques, supportés par des hautes valeurs de "bootstrap" montrent que les familles ERV-9 et HERV-W dérivent de deux vagues d'insertions indépendantes. Ainsi, le(s) élément(s) actif(s) à l'origine de la famille HERV-W est (sont) distinct(s) de celui (ceux) du(des)quel(s) la famille ERV-9 dérive. Dé plus, le PBS de HERV-W utilise probablement un tRNA^{Trp} alors que ERV-9 utilise probablement un tRNA^{Arg}.

Enfin, les membres de la famille HERV-W sont exprimés dans le placenta, alors qu'on ne détecte pas les ARN ERV-9 dans ce tissu.

### FONCTIONS BIOLOGIQUES DE HERV-W

L'expression de HERV-W restreinte au placenta et la longue trame de lecture codant potentiellement pour une enveloppe rétrovirale autorisent à proposer des fonctions biologiques physiologiques dont l'altération pourrait être associée à des pathologies.

L'expression restreinte au placenta suggère que l'expression de gènes rétroviraux et/ou non rétroviraux sous la dépendance des LTR peut être hormone-dépendante. Ces gènes peuvent être adjacents, ou sous la dépendance de LTR isolés. Une pathologie peut alors provenir d'une expression aberrante suite à la réactivation d'un LTR silencieux par divers facteurs : infection virale (par exemple par un membre de la famille des Herpèsvirus) ou activation immune locale. Un polymorphisme au niveau des LTR pourrait aussi favoriser ces événements.

L'enveloppe de HERV-W pourrait jouer un rôle fusogénique, en particulier au niveau de sous-types cellulaires du placenta. Le peptide immunosuppresseur de cette enveloppe pourrait protéger le foetus contre l'agression du système immunitaire maternel. Enfin, par un mécanisme de saturation de récepteurs, l'enveloppe de HERV-W pourrait jouer un rôle protecteur contre les infections rétrovirales exogènes. L'altération de l'immunité cellulaire locale peut découler d'un signal immunostimulateur porté par l'enveloppe. Cet effet peut être lié à une région portant une activité superantigène, ou à la région immunosuppressive qui deviendrait immunostimulatrice à la suite, soit d'un polymorphisme, soit d'un effet-dose (surexpression).

La vérification de ces implications et la compréhension des conséquences liées à une altération des fonctions biologiques des LTR ou de l'enveloppe rétrovirale endogènes peut mener à l'établissement de méthodes de diagnostic ou de suivi :
- d'états de grossesse pathologique ou d'insuccès de grossesse,
- de maladies auto-immunes comme la sclérose en plaques ou la polyarthrite rhumatoïde.

Conformément à la présente invention, il a été découvert, à l'état endogène, un nouveau matériel nucléique, explicité et décrit ci-après, ayant l'organisation d'un rétrovirus, et susceptible d'être corrélé à la sclérose en plaques.

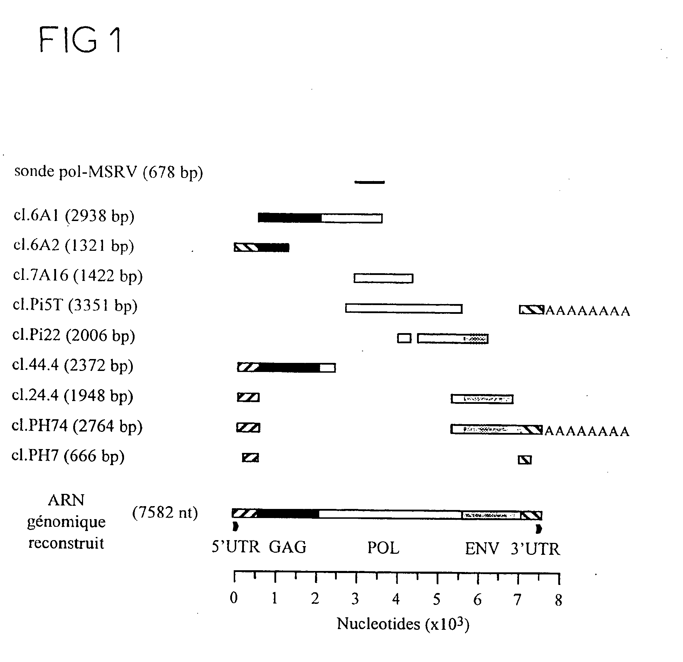

Le matériel nucléique selon la présente invention, sous forme ARNm, représente environ 8 Kb, il est représenté à la Figure 1 et est décrit par SEQ ID NO: 11, et est représenté à la Figure 2 sous forme d'ADN génomique.

Par l'expression "de type rétroviral", on entend la caractéristique selon laquelle le matériel nucléique considéré comprend une ou des séquences nucléotidiques apparentées à l'organisation d'un rétrovirus, et/ou à ses séquences fonctionnelles ou codantes.

Ce matériel nucléique de référence s'apparente à un rétrovirus endogène humain, désigné par l'expression HERV-W. En conséquence, il peut être obtenu par toute technique appropriée de balayage ("screening") de toute banque d'ADN humain, ou d'ADNc placentaire, comme montré ci-après, en particulier avec des amorces ou sondes nucléiques synthétisées pour s'hybrider avec tout ou partie de SEQ ID NO: 11.

La présente invention concerne également tout produit nucléique ou peptidique, obtenu ou dérivé à partir du matériel nucléique de référence, selon SEQ ID NO: 11.

Et l'invention s'intéresse pour terminer aux différentes corrélations pouvant être faites entre le matériel nucléique précité, et/ou ses produits dérivés, avec

La présente invention concerne tout d'abord un matériel nucléique de type génomique rétroviral, à l'état isolé ou purifié, au moins partiellement fonctionnel ou non fonctionnel.

Ce matériel est caractérisé en ce que son génome comprend une séquence nucléotidique de référence choisie dans le groupe incluant les séquences SEQ ID NOs: 1 à 15, et leurs séquences complémentaires

A titre particulier, ce matériel comprend un fragment nucléique inséré entre deux séquences correspondant respectivement à la région LTR et au gène gag de la structure génomique rétrovirale, notamment un fragment nucléique constitué par ou comprenant la séquence SEQ ID NO: 12.

L'invention concerne aussi un matériel nucléique de type rétroviral sous-génomique, constitué par une séquence nucléotidique identique à SEQ ID NO: 11, avec une délétion tel qu'exemplifié par les clones cl.PH74 (SEQ ID NO: 7), cl.PH7 (SEQ ID NO: 8) et cl.Pi5T (SEQ ID NO: 9), cette délétion résultant ou non d'une stratégie d'épissage.

Le matériel nucléique précédemment défini comprend au moins une séquence nucléotidique fonctionnelle codant pour au moins une protéine rétrovirale, et/ou au moins une séquence nucléotidique de régulation.

L'invention concerne aussi toute sonde nucléique de détection d'un matériel nucléique de l'invention, inséré ou non dans un acide nucléique, caractérisée en ce qu'elle est est choisie parmi SEQ ID NO 16-18 et 21-28.

Une telle sonde comprend ou non un marqueur.

L'invention concerne aussi une amorce nucléique pour l'amplification par polymérisation d'un matériel nucléique de l'invention, caractérisée en ce qu'elle est choisie parmi SEQ ID NO. 16 - 18 et 21 - 28.

L'invention concerne aussi tout peptide codé par tout cadre de lecture ouvert appartenant à un fragment nucléotidique, tel que défini précédemment, notamment polypeptide, par exemple oligopeptide formant un déterminant antigénique reconnu par des sera de patients affectés par la sclérose en plaques.

A titre d'exemple, ce polypeptide est codé par un fragment nucléotidique comprenant un cadre de lecture ouvert codant pour une ou des protéines ENV rétrovirales.

Enfin, l'invention concerne :
- l'utilisation d'un matériel nucléique, ou d'un fragment nucléotidique, ou d'un peptide défini ci-dessus, tels que définis précédemment, comme marqueur moléculaire de la sclérose en plaques;
- l'utilisation d'un matériel nucléique, ou d'un fragment nucléotidique, tels que définis précédemment, comme marqueur chromosomique d'une susceptibilité à la sclérose en plaques;
- l'utilisation d'un matériel nucléique, ou d'un fragment nucléotidique, tels que définis précédemment, comme marqueur de proximité d'un gène de susceptibilité à la sclérose en plaques.

Avant de détailler l'invention, différents termes utilisés dans la description et les revendications sont à présent définis:
- par virus humain, on entend un virus susceptible d'infecter ou d'être hébergé par l'être humain,
- compte tenu de toutes les variations et/ou recombinaisons naturelles ou induites, pouvant être rencontrées dans la pratique de la présente invention, les objets de cette dernière, définis ci-dessus et dans les revendications, ont été exprimés en comprenant les équivalents ou dérivés des différents matériels biologiques définis ci-après, notamment las séquences homologues nucléotidiques ou peptidiques,
- le variant d'un virus ou d'un agent pathogène et/ou infectant selon l'invention comprend au moins un antigène reconnu par au moins un anticorps dirigé contre au moins un antigène correspondant dudit virus et/ou dudit agent pathogène et/ou infectant, et/ou un génome dont toute partie est détectée par au moins une sonde d'hybridation, et/ou au moins une amorce d'amplification nucléotidique spécifique dudit virus et/ou agent pathogène et/ou infectant, notamment un génome appartenant à la famille HERV-W, dans des conditions d'hybridation déterminées bien connues de l'homme de l'art,
- selon l'invention, un fragment nucléotidique ou un oligonucléotide ou un polynucléotide est un enchaînement de monomères, ou un biopolymère, caractérisé par la séquence, informationnelle ou non, des acides nucléiques naturels, susceptible de s'hybrider à tout autre fragment nucléotidique dans des conditions prédéterminées, l'enchaînement pouvant contenir des monomères de structures chimiques différentes et être obtenu à partir d'une molécule d'acide nucléique naturelle et/ou par recombinaison génétique et/ou par synthèse chimique ; un fragment nucléotidique peut être identique à un fragment génomique d'un élément de la famille HERV-W considéré par la présente invention, notamment un gène de ce dernier, par exemple pol ou env dans le cas dudit élément ;
- ainsi un monomère peut être un nucléotide naturel d'acide nucléique, dont les éléments constitutifs sont un sucre, un groupement phosphate et une base azotée; dans l'ARN le sucre est le ribose, dans l'ADN le sucre est le désoxy-2-ribose; selon qu'il s'agit de l'ADN ou l'ARN, la base azotée est choisie parmi l'adénine, la guanine, l'uracile, la cytosine, la thymine; ou le nucléotide peut être modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation; au niveau du sucre, la modification peut consister dans le remplacement d'au moins un désoxyribose par un polyamide, et au niveau du groupement phosphate, la modification peut consister dans son remplacement par des esters, notamment choisis parmi les esters de diphosphate, d'alkyl et arylphosphonate et de phosphorothioate,
- par "fonctionnel", on entend la caractéristique selon laquelle une séquence nucléotidique, un matériel nucléique, ou un fragment nucléotidique comprend une "une séquence informationnelle",
- par "séquence informationnelle", on entend toute suite ordonnée de monomères, dont la nature chimique et l'ordre dans un sens de référence, constituent ou non une information fonctionnelle de même qualité que celle des acides nucléiques naturels, par exemple une trame de lecture codant pour une protéine, une séquence régulatrice, un site d'épissage, un site de recombinaison.
- par hybridation, on entend le processus au cours duquel, dans des conditions opératoires appropriées, notamment de stringence, deux fragments nucléotidiques, ayant des séquences suffisamment complémentaires, s'apparient pour former une structure complexe, notamment double ou triple, de préférence sous forme d'hélice,
- une sonde comprend un fragment nucléotidique synthétisé notamment par voie chimique ou polymérisation, ou obtenu par digestion ou coupure enzymatique d'un fragment nucléotidique plus long, comprenant au moins six monomères, avantageusement de 10 à 100 monomères, de préférence 10 à 30 monomères, et possédant une spécificité d'hybridation dans des conditions déterminées ; de préférence, une sonde possédant moins de 10 monomères n'est pas utilisée seule, mais l'est en présence d'autres sondes de taille aussi courte ou non ; dans certaines conditions particulières, il peut être utile d'utiliser des sondes de taille supérieure à 100 monomères ; une sonde peut notamment être utilisée à des fins de diagnostic et il s'agira par exemple de sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection peut être marquée au moyen d'un marqueur choisi notamment parmi les isotopes radioactifs, des enzymes notamment choisis parmi la peroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues de l'homme de l'art, et notamment les techniques dites "DOT-BLOT", "SOUTHERN BLOT", "NORTHERN BLOT" qui est une technique identique à la technique "SOUTHERN BLOT" mais qui utilise de l'ARN comme cible, la technique SANDWICH; avantageusement, on utilise la technique SANDWICH dans la présente invention, comprenant une sonde de capture spécifique et/ou une sonde de détection spécifique, étant entendu que la sonde de capture et la sonde de détection doivent présenter une séquence nucléotidique au moins partiellement différente,
- toute sonde selon la présente invention peut s'hybrider in vivo ou in vitro sur l'ARN et/ou sur l'ADN, pour bloquer les phénomènes de réplication, notamment traduction et/ou transcription, et/ou pour dégrader ledit ADN et/ou ARN,
- une amorce est une sonde comprenant au moins six monomères, et avantageusement de 10 à 30 monomères, possédant une spécificité d'hybridation dans des conditions déterminées, pour l'initiation d'une polymérisation enzymatique, par exemple dans une technique d'amplification telle que la PCR (Polymerase Chain Reaction), dans un procédé d'élongation, tel que le séquençage, dans une méthode de transcription inverse ou analogue,
- deux séquences nucléotidiques ou peptidiques sont dites équivalentes ou dérivées l'une par rapport à l'autre, ou par rapport à une séquence de référence, si fonctionnellement les biopolymères correspondants peuvent jouer sensiblement le même rôle, sans être identiques, vis-à-vis de l'application ou utilisation considérée, ou dans la technique dans laquelle elles interviennent ; sont notamment équivalentes deux séquences obtenues du fait de la variabilité naturelle au sein d'un même individu, ou de la diversité naturelle d'un individu à un autre au sein d'une même espèce, notamment mutation spontanée de l'espèce à partir de laquelle elles ont été identifiées, ou induite, ainsi que deux séquences homologues, l'homologie étant définie ci-après,
- par "variabilité", on entend toute modification, spontanée ou induite d'une séquence, notamment par substitution, et/ou insertion, et/ou délétion de nucléotides et/ou de fragments nucléotidiques, et/ou extension et/ou raccourcissement de la séquence à l'une au moins des extrémités; une variabilité non naturelle peut résulter des techniques de génie génétique utilisées, par exemple du choix des amorces de synthèse, dégénérées ou non, retenues pour amplifier un acide nucléique; cette variabilité peut se traduire par des modifications de toute séquence de départ, considérée comme référence, et pouvant être exprimées par un degré d'homologie par rapport à ladite séquence de référence,
- l'homologie caractérise le degré d'identité de deux fragments nucléotidiques ou peptidiques comparés ; elle se mesure par le pourcentage d'identité qui est notamment déterminé par comparaison directe de séquences nucléoditiques ou peptidiques, par rapport à des séquences nucléotidiques ou peptidiques de référence,
- ce pourcentage d'identité a été spécifiquement déterminé pour les fragments nucléotidiques, notamment clones relevant de la présente invention, et provenant d'un même individu; à titre d'exemple non limitatif, le plus faible pourcentage d'identité observé entre les différents clones d'un même individu (cf SEQ ID NOs: 13 et 14) est d'au moins 90% et le plus faible pourcentage d'identité observé entre les différents clones de deux individus est d'au moins 80%,
- tout fragment nucléotidique est dit équivalent ou dérivé d'un fragment de référence, s'il présente une séquence nucléotidique équivalente à la séquence du fragment de référence ; selon la définition précédente, sont notamment équivalents à un fragment nucléotidique de référence :
   (a) tout fragment susceptible de s'hybrider au moins partiellement avec le complément du fragment de référence,
   (b) tout fragment dont l'alignement avec le fragment de référence conduit à mettre en évidence des bases contigües identiques, en nombre plus important qu'avec tout autre fragment provenant d'un autre groupe taxonomique,
   (c) tout fragment résultant ou pouvant résulter de la variabilité naturelle au sein d'un même individu, et de la diversité naturelle d'un individu à un autre dans la même espèce, à partir desquels il est obtenu,
   (d) tout fragment pouvant résulter des techniques de génie génétique appliquées au fragment de référence,
   (e) tout fragment, comportant au moins huit nucléotides contigus, codant pour un peptide homologue ou identique au peptide codé par le fragment de référence,
   (f) tout fragment différent du fragment de référence, par insertion, délétion, substitution d'au moins un monomère, extension, ou raccourcissement à l'une au moins de ses extrémités ; par exemple, tout fragment correspondant au fragment de référence, flanqué à l'une au moins de ses extrémités par une séquence nucléotidique ne codant pas pour un polypeptide,
- par séquence nucléotidique, partielle ou totale, d'un matériel nucléique de référence, on entend également toute séquence associée par co-encapsidation, ou par coexpression, ou recombinée avec ledit matériel nucléique de référence,
- par polypeptide, on entend notamment tout peptide d'au moins deux acides aminés, notamment oligopeptide, protéine, extrait, séparé, ou substantiellement isolé ou synthétisé, par l'intervention de la main de l'homme, notamment ceux obtenus par synthèse chimique, ou par expression dans un organisme recombinant,
- par polypeptide codé de manière partielle par un fragment nucléotidique, on entend un polypeptide présentant au moins trois acides aminés codés par au moins neuf monomères contigus compris dans ledit fragment nucléotidique,
- un acide aminé est dit analogue à un autre acide aminé, lorsque leur caractéristiques physico-chimiques respectives, telles que polarité, hydrophobicité, et/ou basicité, et/ou acidité, et/ou neutralité, sont sensiblement les mêmes ; ainsi, une leucine est analogue à une isoleucine,
- tout polypeptide est dit équivalent ou dérivé d'un polypeptide de référence, si les polypeptides comparés ont sensiblement les mêmes propriétés, et notamment les mêmes propriétés antigéniques, immunologiques, enzymologiques et/ou de reconnaissance moléculaire ; est notamment équivalent à un polypeptide de référence :
   (a) tout polypeptide possédant une séquence dont au moins un acide aminé a été substitué par un acide aminé analogue,
   (b) tout polypeptide ayant une séquence peptidique équivalente, obtenue par variation naturelle ou induite dudit polypeptide de référence, et/ou du fragment nucléotidique codant pour ledit polypeptide,
   (c) un mimotope dudit polypeptide de référence,
   (d) tout polypeptide dans la séquence duquel un ou plusieurs acides aminés de la série L sont remplacés par un acide aminé de la série D, et vice versa,
   (e) tout polypeptide dans la séquence duquel on a introduit une modification des chaînes latérales des acides aminés, telle que par exemple une acétylation des fonctions amines, une carboxylation des fonctions thiol, une estérification des fonctions carboxyliques,
   (f) tout polypeptide dans la séquence duquel une ou des liaisons peptidiques ont été modifiées, comme par exemple les liaisons carba, rétro, inverso, rétro-inverso, réduites, et méthylène-oxy,
   (g) tout polypeptide dont au moins un antigène est reconnu par un anticorps dirigé contre un polypeptide de référence,
- le pourcentage d'identité caractérisant l'homologie de deux fragments peptidiques comparés est selon la présente invention d'au moins 80% et de préférence au moins 90%.

Les expressions d'ordre utilisées dans la présente description et les revendications, telles que "première séquence nucléotidique" ne sont pas retenues pour exprimer un ordre particulier, mais pour définir plus clairement l'invention.

Par détection d'une substance ou agent, on entend ci-après aussi bien une identification, qu'une quantification, ou une séparation ou isolement de ladite substance ou dudit agent.

L'invention sera mieux comprise à la lecture de la description détaillée qui va suivre faite en référence aux Figures annexées dans lesquelles :
- la Figure 1 représente, d'une part, l'organisation du matériel rétroviral endogène découvert selon la présente invention, sous la forme d'un ARNm génomique putatif, et, d'autre part, la localisation des clones mise en oeuvre selon la présente invention, par rapport à cette organisation; les échelles de longueur sont exprimées en Kb ; les régions flanquantes (5' UTR et 3' UTR) sont indiquées dans des boîtes hachurées ; les régions répétées dans ces deux régions flanquantes sont indiquées par des flèches noires ; les régions correspondant aux gènes gag, pol, et env, sont indiqués respectivement, en noir, blanc et gris ; le positionnement de la sonde Ppol-MSRV est indiqué ;
- la Figure 2 représente une possibilité d'organisation génétique (ADN), illustrée par le clone RG083M05, et une stratégie d'épissage liant à cette séquence, les clones expérimentaux (ARNm) ; cette figure montre également les sites d'épissage observés par référence à l'organisation rétrovirale ; sur cette figure sont en outre indiqués :
   la localisation des sondes utilisées (Pgag-LB19, Ppro-E, Ppol-MSRV et Penv-C15) ;
   les sites donneurs (DS1 et DS2) et accepteurs (AS1 à AS3) d'épissage ;
   les séquences provenant du clone RG083M05, dans les boites en minuscules, et les séquences dérivant des clones expérimentaux placentaires (ARNm), dans les boites en majuscule ;
   les ORFs putatives (ORF1, ORF2 et ORF3) ; et
   un insert de 2 Kb présent sous forme ADN mais non détecté sous forme ARN, représenté sous forme de hachures verticales.

   Les autres conventions utilisées dans cette figure sont les mêmes que celles de la Figure 1.

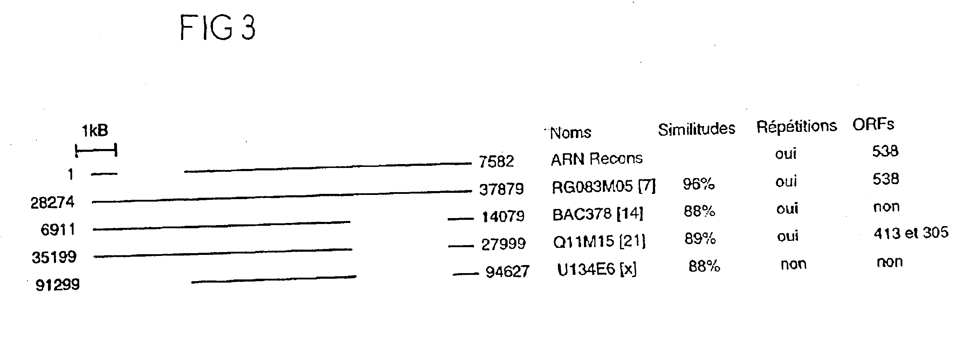

- la Figure 3 donne une représentation de clones génomiques (ADN) correspondant aux clones d'ADNc isolés ; sur cette figure, sont indiqués :
   le pourcentage de similitude vis-à-vis de l'ARN génomique reconstruit (ARN Recons) ;
   la présence de séquences répétées à chaque extrémité de ces génomes (répétitions) ; et
   la présence et la taille des trames de lecture ouverte (ORFs).

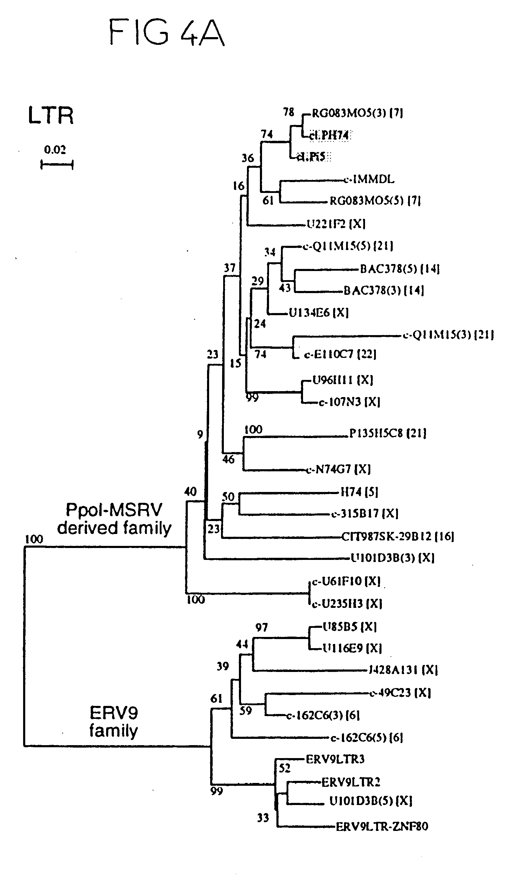

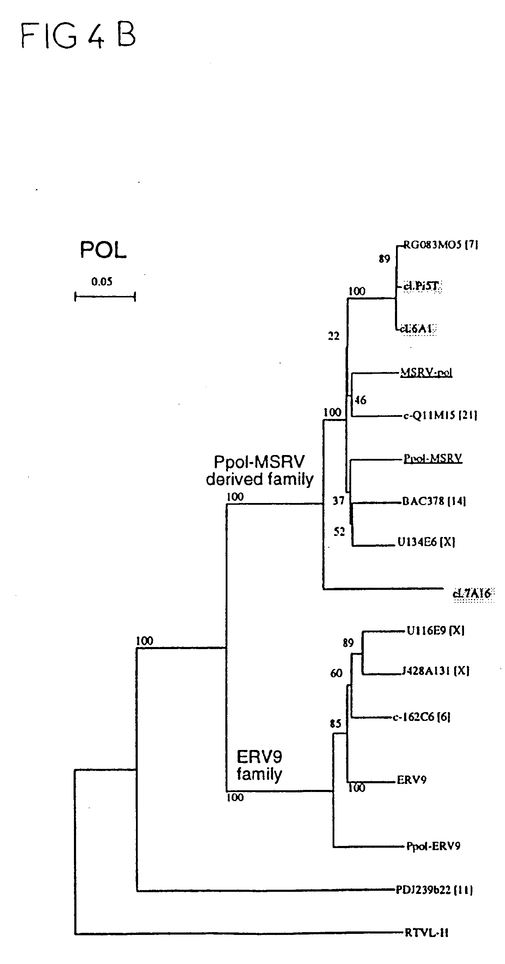

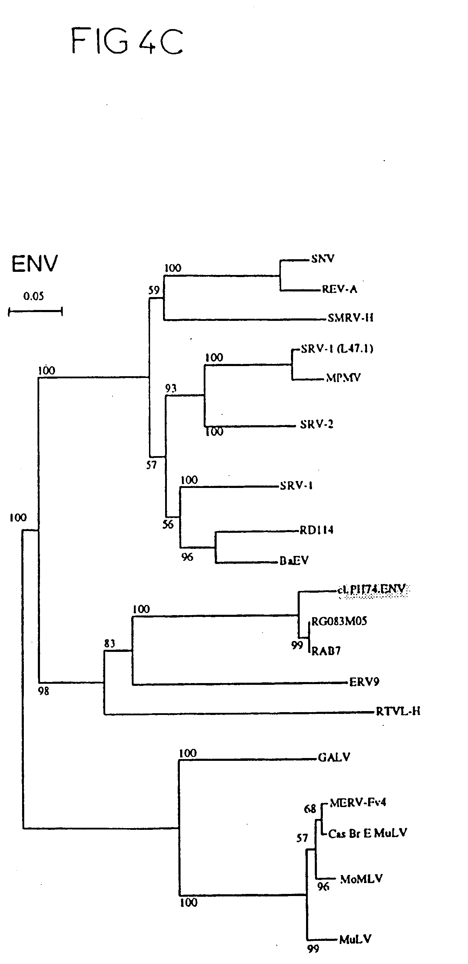

- la Figure 4 représente une analyse phylogénique identifiant la famille HERV-W.

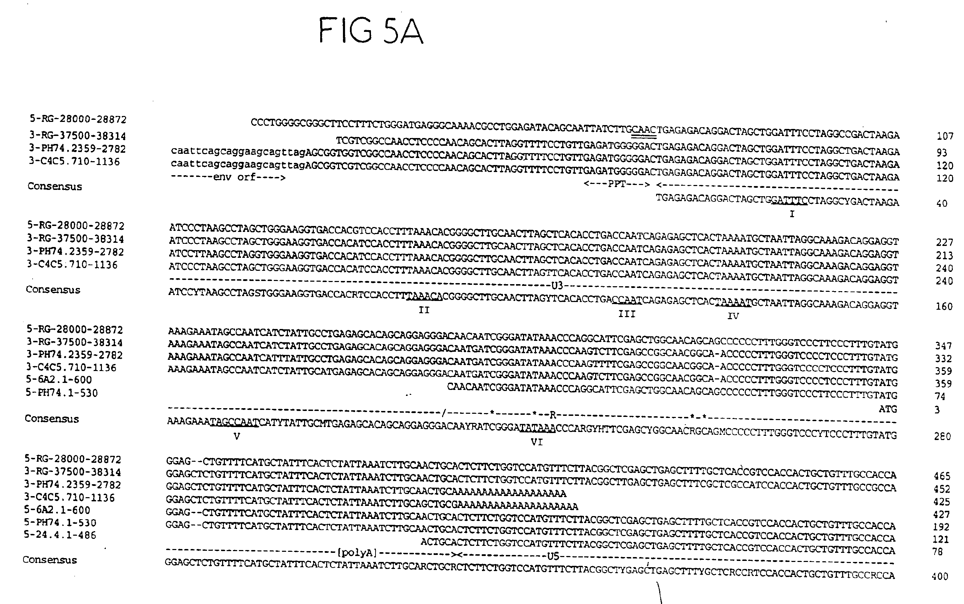

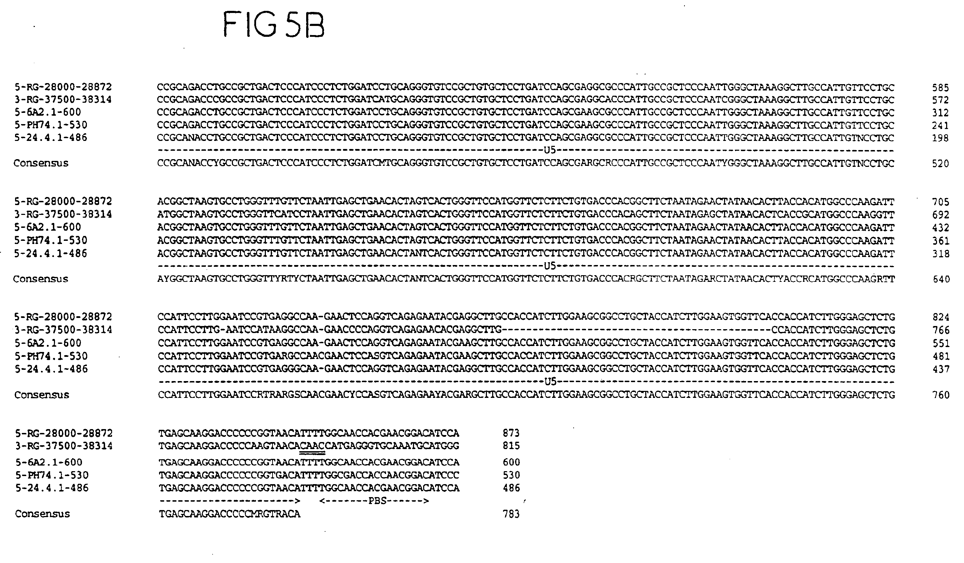

- la figure 5 représente l'alignement des régions flanquantes 5' et 3' du clone RG083M05 avec les régions 5' et/ou 3' terminales de certains clones placentaires ; le tandem CAAC flanquant les LTR 3' et 5' est souligné doublement sous les séquences d'ADN, la séquence LTR consensus de 783 pb (paires de bases) est indiqué au bas de l'alignement ; le PPT en amont de l'extrémité 5' de LTR et le PBS en aval de l'extrémité 3' de LTR sont indiqués ; les régions U3R et U5 sont indiquées ; les sites correspondant à la fixation du facteur de transcription sont soulignés et numérotés de 1 à 6 ; la région -73 à 284 correspond à la séquence évaluée en "CAT assay" ; * correspond à des sites putatifs de "capping" ; [polyA] indique le signal de polyadénylation.

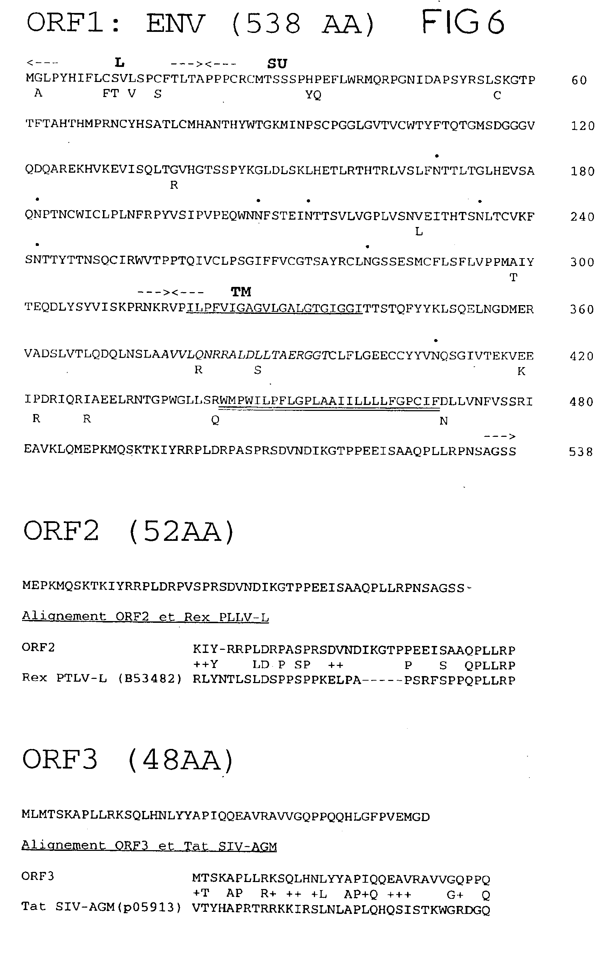

- la Figure 6 représente une séquence putative d'un polypeptide d'enveloppe (ORF1) de HERV-W obtenu à partir de 3 clones d'ADNc placentaires différents ; le peptide leader (L), la protéine de surface (SU) et la protéine transmembranaire (TM) sont indiquées par des flèches ; le peptide de fusion hydrophobe et la région carboxy transmembranaire sont soulignés par un trait simple et un trait double, respectivement ; la région d'immunosuppression est signalée en italiques ; les sites potentiels de glycosylation sont indiqués par des points ; les acides aminés divergents sont indiqués sur la ligne inférieure ; la figure 6 présente également les trames de lecture ouverte correspondant à ORF2 et ORF3 tels que décrits à la Figure 2, et plus particulièrement leurs homologies avec les gènes de régulation rétroviraux.

Le matériel nucléique précédemment explicité a été découvert et caractérisé au terme du protocole expérimental décrit ci-après, étant entendu que ce protocole ne saurait limiter la portée de la présente invention et des revendications en annexe.

### Exemple 1

### Isolement et séquençage de fragments d'ADNc chevauchants

Les informations concernant l'organisation de HERV-W ont été obtenues en testant une banque d'ADNc placentaire (Clontech cat#HL5014a) avec les sondes Ppol-MSRV (SEQ ID NO: 29) et Penv-C15 (SEQ ID NO: 31) (cf Exemple 8), et en pratiquant ensuite une technique de "gene walking" à l'aide des nouvelles séquences obtenues. Les expériences ont été mises en oeuvre en se référant aux préconisations du fournisseur de la banque. Des amplifications PCR sur ADN ont également été exploitées pour comprendre cette organisation.

Un certain nombre de clones ont été sélectionnés et séquencés, cf Figure 1:
- Clone cl.6A2 (SEQ ID NO: 1) : région 5' non traduite de HERV-W et une partie de gag
- Clone c1.6A1 (SEQ ID NO: 2): gag et une partie de pol
- Clone cl.7A16 (SEQ ID NO: 3): Région 3' de pol
- Clone cl.Pi22 (SEQ ID NO: 4): région 3' de pol et début de env
- Clone c1.24.4 (SEQ ID NO: 5) : ARN épissé comprenant une partie de la région 5' non traduite de HERV-W, la fin de pol et la région 5' de env
- Clone cl.C4C5. (SEQ ID NO: 6) : fin de env et région 3' non traduite de HERV-W
- Clone cl.PH74 (SEQ ID NO: 7) : ARN sous-génomique : région 5' non traduite de HERV-W, fin de pol, env, et région 3' non traduite de HERV-W
- Clone cl.PH7 (SEQ ID NO: 8) : ARN multi-épissé : région 5' non traduite de HERV-W, fin de env et région 3' non traduite de HERV-W.
- Clone cl.Pi5T (SEQ ID NO: 9) : gène pol partiel et région U3-R
- Clone cl.44.4 (SEQ ID NO: 10) : région R-U5, gène gag et gène pol partiel.

A l'aide de ces clones, en procédant à des alignements de séquences, un modèle de séquence totale de HERV-W a été élaboré. Les ARN épissés ont été mis en évidence ainsi que les sites potentiels donneurs et accepteurs d'épissage. L'ensemble de ces informations est montré à la Figure 2. Par étude de similitude avec des rétrovirus existants, les entités LTR, gag, pol et env ont été définies.

L'organisation génétique putative de HERV-W sous forme ARN est la suivante (SEQ ID NO: 11):
gène 1..7582
Localisation des clones sur la séquence ARN génomique reconstruite
   cl.6A2 (1321 pb) 1-1325 ;
   cl.PH74 (535+2229= 2764 pb) 72-606 et 5353-7582;
   cl.24.4 (491+1457= 1948 pb); 115-606 et 5353-6810;
   cl.44.4 (2372 pb) 115-2496 ;
   cl.PH7 (369+297= 666pb) 237-606 et 7017-7313;
   cl.6A1 (2938 pb) 586-3559.;
   cl.Pi5T (2785+566= 3351 pb) 2747-5557 et 7017-7582;
   cl.7A16 (1422 pb) 2908-4337;
   cl.Pi22 (317+1689 = 2006 pb) 3957-4273 et 4476-6168;
   cl.C4C5 (1116 pb) 6467-7582

- 5'LTR: 1..120
/note="R of 5'LTR (extrémité 5'
incertaine"
121..575
/note="U5 of 5'LTR"
- divers: 579..596
/note="PBS primer binding site pour tRNA-W"
- divers: 606
/note="jonction d'épissage (site donneur
d'épissage ATCCAAAGTG-GTGAGTAATA et site
accepteur d'épissage CTTTTTTCAG-ATGGGAAACG clone RG083MO5, GenBank accession
AC000064)"
- divers: 5353
/note="site accepteur d'épissage pour
l'ORF1 (env)"
- divers: 5560
/note="site donneur d'épissage"
- ORF: 5581..7194
/note="ORF1 env 538 AA"
/produit-="enveloppe"
- divers: 7017
/note="site accepteur d'épissage pour ORF2 et ORF3"
- ORF: 7039..7194
/note="ORF2 52 AA"
- ORF: 7112..7255
/note="ORF3 48 AA"
- divers: 7244..7254
/note="PPT polypurine tract"
- 3'LTR: 7256..7582
/note-="U3-R of 3' LTR (jonction U3-R indéterminée)
- divers: 7563..7569
signal de polyadénylation

### Exemple 2 :

### Identification de clones génomiques (ADN) correspondant aux clones d'ADN isolés

Une interrogation "blastn" sur plusieurs bases de données, à l'aide du génome reconstruit, montre qu'il existe une quantité importante de séquences apparentées dans le génome humain. Environ 400 séquences ont été identifiées dans GenBank et plus de 200 séquences dans la banque EST, la plupart en anti-sens. Les 4 séquences les plus significatives en taille et en similitude, illustrées sur la figure 3, sont les clones génomiques (ADN) suivants :
le clone humain RG083M05 (gb AC000064) dont la localisation chromosomique est 7q21-7q22,
le clone humain BAC378 (gb U85196, gb AE000660) correspondant au locus alpha delta du récepteur des cellules T, localisé en 14q11-12,
le cosmide humain Q11M15 (gb AF045450) correspondant à la région 21q22.3 du chromosome 21,
le cosmide U134E6 (embl Z83850) sur le chromosome Xq22.

La localisation des régions alignées pour chacun des clones est indiquée et l'appartenance à un chromosome est indiquée entre crochets. Le pourcentage de similitude (sans les larges délétions) entre les 4 séquences et l'ARN génomique reconstruit est indiqué, ainsi que la présence de séquences répétées à chaque extrémité du génome et la taille des plus grandes trames de lecture (ORF). Des séquences répétées sont trouvées aux extrémités de 3 de ces clones. La séquence reconstruite est contenue intégralement à l'intérieur du clone RG083M05 (9,6 Kb) et présente une similitude de 96%. Cependant le clone RG083M05 présente une insertion de 2 Kb située immédiatement en aval de la région 5' non traduite (5' UTR). Cette insertion est également trouvée dans deux autres clones génomiques qui présentent une délétion de 2,3 Kb immédiatement en amont de la région 3' non traduite (3' UTR). Aucun clone ne contient les trois trames de lecture (ORFs) gag, pol et env fonctionnelles. Le clone RG083M05 montre une ORF de 538 acides aminés (AA) correspondant à une enveloppe entière. Le cosmide Q11M15 contient deux grandes ORFs contigües de 413 AA (trame 0) et 305 AA (trame +1) correspondant à une polyprotéine pol tronquée.

### Exemple 3

### Analyse phylogénique

Une analyse phylogénique a été réalisée au niveau des acides nucléiques sur 11 sous-régions différentes de l'ARN génomique reconstruit, et au niveau protéique sur 2 sous-régions différentes de env. Tous les arbres obtenus présentent la même topologie quelle que soit la région étudiée. Ceci est illustré à la Figure 4 au niveau des acides nucléiques dans les régions LTR et pol les plus conservées entre les séquences obtenues et ERV-9 et RTLV-H. Les arbres montrent clairement que les séquences expérimentales décrivent une nouvelle famille distincte de ERV-9 et très distincte de RTLV-H comme souligné par l'analyse en "bootstrap". Ces séquences sont trouvées sur plusieurs chromosomes, notamment les chromosomes 5, 7, 14, 16, 21, 22, et X avec une forte concentration apparente de LTR sur le chromosome X.

La comparaison au niveau protéique entre les régions les plus conservées des protéines rétrovirales env montre que la famille HERV-W est plus proche des rétrovirus simiens de type D et des rétrovirus de la réticuloendothéliose aviaire que les rétrovirus mammifères de type C.

Ceci suggère une structure génomique chimère C/D.

### Exemple 4

### Identification des éléments LTR, PPT et PBS

La séquence reconstruite (ARN) est contenue intégralement à l'intérieur du clone génomique RG083M05 (9,6 Kb) et présente une similitude de 96 % avec deux régions discontinues de ce clone qui contient également des régions répétées à chaque extrémité. L'alignement des séquences expérimentales correspondant aux régions 5' et 3' de l'ARN génomique reconstruit avec l'ADN du clone RG083M05 [5'(5-RG-28000-28872) et 3'(3-RG-37500-38314)] a permis de déduire une séquence LTR et d'identifier des éléments caractéristiques des rétrovirus, notamment ceux impliqués dans la transcription inverse, à savoir PBS en aval du LTR 5' et le PPT en amont du LTR 3' (cf Figure 5). On remarque que l'élément U3 est extrêmement court en comparaison de celui observé chez les rétrovirus de type C des mammifères, et est comparable en taille à la région U3 généralement décrite chez les rétrovirus de type D et les rétrovirus aviaire. La région correspondant aux bases 2364 à 2720 du clone cl.PH74 (SEQ ID NO: 7) a été amplifiée par PCR et sous-clonée dans le vecteur pCAT3 (Promega) afin de réaliser l'évaluation de l'activité promotrice. Une activité significative a été trouvée dans des cellules HeLa par la méthode dite du "CAT assay" montrant la fonctionnalité de la séquence promotrice du LTR.

La région PBS est homologue au PBS des rétrovirus aviaires.

### Exemple 5

### organisation génétique et régulation de l'expression

### Organisation sous forme ADN

Des amplifications PCR ont été réalisées sur des clones HERV-W entiers récupérés sur banque génomique humaine (voir exemple 1 pour le mode d'obtention), en utilisant les couples d'oligonucléotides suivants :
U5 4992 (SEQ ID NO: 16), GAG 4619 (SEQ ID NO: 17)
GAG 4782 (SEQ ID NO: 18), POL 3167 (SEQ ID NO: 19)
POL 3390 (SEQ ID NO: 20), POL 5144 (SEQ ID NO: 21)
POL 5145 (SEQ ID NO: 22), U5 4991 (SEQ ID NO: 23).

Les PCR sont réalisées dans les conditions suivantes :
Oligonucléotides à la concentration de 0,33 microMolaire
Tampon TAQ polymérase Boerhinger 1X
0,5 unité de TAQ polymérase Boerhinger
Mélange de dNTP à 0,25 mM chacun
0,5 mg d'ADN humain
Volume final 100 ml
Conditions de PCR (95°C, 5 min) x 1, (95°C, 30 sec + 54°C, 30 sec + 72°C 3 min) x 35.

Les produits de PCR ont ensuite été déposés sur gel d'agarose 1% pour être analysés après migration. L'ensemble des PCR donne des fragments d'amplification de taille attendue, excepté pour la PCR LTR-4991--gag-4619 qui donne un fragment de taille supérieure d'environ 2 Kb par rapport à la taille attendue (déduite à partir des cDNA de la banque placentaire). La reconstitution de HERV-W sous forme ADN endogène représente donc une entité d'environ 10 Kb.

Après clonage, séquençage et analyse de la PCR-4992 gag-4619, on constate la présence d'une région d'insertion entre LTR et gag de SEQ ID NO: 12 (clone cl.6A5). Cette région ne correspond pas à une région traditionnelle non traduite d'un rétrovirus : pas de région Ψ ni de PBS.

Les produits de PCR pol-3390, pol-5144 ont été également clonés et deux des clones obtenus ont été séquencés. Le résultat de ces séquences est donné par les clones cl.7A20 (SEQ ID NO: 13) et cl.7A21 (SEQ ID NO: 14). La comparaison de ces deux séquences nucléotidiques donne un score de 90% d'homologie pour la région concernée, montrant ainsi la variabilité de HERV-W chez un même individu.

HERV-W sous forme ADN est proposé la Figure 2.

Organisation générale : processus de transcription

Les différents clones ADNc étant obtenus, des résultats acquis en PCR sur ADN, on déduit :
- une organisation ADN de 10 Kb possédant une séquence d'insertion de 2 Kb entre LTR et gag.

Le résultat de PCR sur ADN montrant la présence d'un insert de 2 Kb entre les régions LTR et gag suggère que les ADNc isolés dans le placenta proviennent de l'expression d'un génome de type RG083M05.
- une organisation ARN de 8 Kb résultant d'une transcription de 10 Kb suivie d'un épissage entre LTR et gag permettant de restaurer une continuité RF (Région Flanquante) 5' gag, et donnant ainsi un ARN de 8 Kb tel que mis en évidence en Northern Blot.

Les sondes gag (Pgag-LB19, SEQ ID NO: 30) et protéase (Ppro-E, SEQ ID NO: 32) révèlent un ARN de taille voisine à 8 Kb, la sonde Penv-C15 (SEQ ID NO: 31) révèle en plus un ARN voisin de 3,1 Kb. Deux sondes définies dans la région 5' non traduite, obtenues par le criblage de la banque cDNA relaté ci-dessus (sonde P5'-gag-cl.6A2 dérivée du clone cl.6A2 et sonde P5'-env-cl.24.4 dérivée du clone cl.24.4) révèlent les deux précédents ARN et un ARN d'environ 1,3 Kb. Cette distribution des ARNs est typique de transcrits de rétrovirus complexes : un ARN génomique codant pour gag-pro-pol, un ARN sous-génomique codant pour l'enveloppe, et un/des ARN multi-épissé(s) codant potentiellement pour des gènes de régulation.

La demie vie d'un tel ARN (LTR-R-U5-Insertion-GAG-POL-ENV-U3-R-HERV-W) est vraisemblablement très courte, car aucun ARN de 10 Kb n'est détecté en Northern Blot. Par analyse et comparaison de séquences, les sites potentiels donneurs (DS1 et DS2) et accepteurs (AS1 à AS3) d'épissage ont été définis et décrits dans la Figure 2.

### Exemple 6

### Transcription dans des tissus sains

Différents tissus humains sains ont été testés par une technique de Northern Blot (Human Multiple Tissue Northern Blot, Clontech cat# 7760-1), à l'aide des sondes Ppol-MSRV (SEQ ID NO: 29), Pgag-LB19 (SEQ ID NO: 30), Penv-C15 (SEQ ID NO: 31), Ppro-E (SEQ ID NO: 32), P5'-gag-cl.6A2 et P5'-env-cl.24.4, marquées comme décrit dans l'exemple 1. Les expériences ont été réalisées en suivant les recommandations des fabricants, et les autoradiographies ont été exposées 5 jours. L'analyse des résultats révèle des produits de transcription uniquement dans le placenta, et dans aucun des autres tissus humains testés (coeur, cerveau, poumon, foie, muscle squelettique, rein et pancréas).

Par une technique de Dot Blot ARN (Clontech : Human RNA Master Blot Cat# 7770-1), et en utilisant le protocole expérimental préconisé par le fabricant, une quarantaine d'autres tissus, dont des tissus foetaux, ont été testés : seul le placenta donne une réponse spécifique après hybridation avec les sondes Pgag-LB19 (SEQ ID NO: 30) et Penv-C15 (SEQ ID NO: 31).

On constate qu'un signal est observé dans le rein en Dot-Blot ARN, ce qui est infirmé par l'analyse en Northern Blot.

### Exemple 7

### Identification d'un ARNm codant pour une enveloppe et les moyens de le détecter spécifiquement

Le criblage d'une banque d'ADNc placentaire à l'aide d'une sonde définie dans la région 5' non traduite a permis d'isoler un ADNc défini par une région 5' non traduite (5' NTR), une jonction d'épissage, une séquence codante, une région 3' non traduite (3' NTR) et une queue polyadénylée, cl.PH74 (SEQ ID NO: 7). Ce clone correspond à un ARN épissé codant pour une enveloppe. Par comparaison de séquences entre ce cDNA et le modèle de HERV-W endogène proposé selon Figure 2, on identifie une jonction d'épissage sur l'ARNm, jonction d'épissage mettant en continuité la région 5' NTR et le gène env, conduisant à l'élaboration d'un ARN sous génomique épissé codant pour le gène d'enveloppe. Ces informations ont permis de définir un oligonucléotide spécifique de cet ARNm, en choisissant une localisation située sur le site d'épissage (Oligo 5307, selon SEQ ID NO: 24).

La mise en évidence de cette région de jonction permet d'établir un procédé de discrimination entre ARN et ADN rétroviral endogène, en utilisant dans une PCR un oligonucléotide défini sur cette région de jonction, notamment un oligonucléotide choisi dans le gène env (Oligo 4986, selon SEQ ID NO: 25).

Les PCR sont réalisées dans les conditions suivantes :
Oligonucléotides à la concentration de 0,33 microMolaire
Tampon TAQ polymérase Boerhinger 1X
0,5 unité de TAQ polymérase Boerhinger
Mélange de dNTP à 0,25 mM chacun
0,5 mg d'ADN humain
Volume final 100 ml

Sur 10 ADN différents testés, ce type de PCR n'a pas permis d'obtenir de produits d'amplification. Par contre sur ADNc issu d'ARN placentaire ou de cellules exprimant HERV-W, cette PCR donne un produit d'amplification. Ce résultat confirme donc la nature spécifiquement ARN de ce fragment sous-génomique.

### Exemple 8

### Identification de séquences codantes, contenues dans un ARNm spécifique

La stratégie d'épissage décrite dans l'exemple 5 est compatible avec la présence de trois trames de lecture ORF1 (SEQ ID NO: 33), ORF2 (SEQ ID NO: 34) et ORF3 (SEQ ID NO: 35) (cf Figure 6).

Le criblage d'une banque d'ADNc placentaire a permis d'isoler un ADNc (SEQ ID NO: 7, cl.PH74) défini par une région 5' non traduite (5' NTR), une jonction d'épissage, une séquence codante, une région 3' non traduite (3' NTR) et une queue polyadénylée. La séquence codante est de 538 acides aminés (SEQ ID NO: 33). Les analyses effectuées sur banques de données permettent de mettre en évidence des caractéristiques d'une enveloppe rétrovirale complète. : début de traduction d'une polyprotéine d'enveloppe, d'un peptide leader fortement hydrophobe d'environ 21 acides aminés, d'une protéine de surface SU, d'une protéine transmembranaire TM. Ces deux entités protéiques présentent différents sites potentiels de glycosylation. Au sein de la protéine TM, on identifie une région immunosuppressive.

22 pb et 95 pb en amont du site accepteur d'épissage, on a trouvé respectivement deux codons d'initiation susceptibles de diriger la synthèse de 52 AA (ORF2, SEQ ID NO: 34) et de 48 AA (ORF3, SEQ ID NO: 35). ORF2 consiste en une partie de l'extrémité carboxy-terminale de env et ORF3 correspond à une traduction différente mais chevauchante.

Aucune homologie significative n'a été retrouvée par interrogation "blast". Cependant une interrogation LFASTA dans une sous-banque de donnée limitée aux Rétroviridae, ORF2 et ORF3 ont montré un pourcentage d'idendité de 35 % avec, respectivement, Rex du virus T-lymphotrope humain et primate, et avec Tat du virus simien de l'immunodéficience.

### Exemple 9

### Complexité de la famille HERV-W

Le nombre de copies présentes dans le génome humain de chacune des séquences est évalué par une technique de Dot Blot, à l'aide des sondes Pgag-LB19 (SEQ ID NO: 30), Ppro-E (SEQ ID NO: 32) et Penv-C15 (SEQ ID NO: 31).

Chacune des sondes est dénaturée et déposée sur une membrane Hybond N+ à raison de 2,5, 5, 10, 25, 50, 100 pg par dépôt. 0,5 mg d'ADN humain sont également déposés sur la même membrane. Les membranes sont séchées 2 heures sous vide à 80°C. Les membranes sont ensuite hybridées avec la sonde déposée. Les techniques de marquage des sondes, d'hybridation et de lavage des membranes sont les mêmes que pour le Southern Blot. Après autoradiographie des membranes, on constate des niveaux d'intensité de signal proportionnels aux dépôts sur membrane. Après découpage des zones d'hybridation, un comptage en scintillation est réalisé. Par comparaison entre la gamme de dilution de la sonde déposée sur membrane et le résultat obtenu avec l'ADN humain, on peut évaluer le nombre de copie par génome haploïde de chacune des régions couvertes par les sondes :
- le nombre de gag endogène est évalué de 56 à 112 copies (76)
- le nombre de protéase endogène est évalué de 166 à 334 copies (260)
- le nombre de env endogène est évalué inférieur à 52 copies (13).

Le criblage de 10⁶ clones d'une banque d'ADN placentaire humain (Clontech cat# Hl5014b) a permis de dénombrer 144 clones reconnus par la sonde Pgag-LB19, et 64 clones reconnus par la sonde Penv-C15. 13 clones hybridés conjointement par les sondes Penv-C15 et Pgag-LB19 ont été isolés, confirmant la présence de plusieurs copies d'un génome possédant à la fois gag et env, sans considération de fonctionnalité.

Le matériel nucléique, les séquences nucléotidiques, et les peptides ou protéines éventuellement exprimées par lesdits matériels et séquences, peuvent être utilisés pour détecter, prévoir, traiter et suivre et sclérose en plaques.

En effet, les données objectives et expérimentales permettent de relier rétrovirus et sclérose en plaques :
(1) des mécanismes communs sont mis en oeuvre dans les pathologies rétrovirales et dans des maladies auto-immunes (présence d'auto-anticorps, de complexes immuns, infiltration cellulaire de certains tissus, troubles neurologiques).
(2) des désordres pathologiques comparables à certaines maladies auto-immunes apparaissent lors des infections par les rétrovirus HIV et HTLV (syndrome de Sjögren, lupus érythémateux disséminé, arthrite rhumatoïde...).
(3) une activité transcriptase inverse a été détectée et des particules de type rétroviral ont été observées dans les surnageants de cultures cellulaires de patients atteints de sclérose en plaques (Perron et coll, Res. Virol. 1989; 140: 551-561 / Lancet 1991; 337: 862-863 / Res. Virol. 1992; 143: 337-350) ou de polyarthrite rhumatoïde.
(4) des pathologies animales inflammatoires chroniques ou auto-immunes sont liées aux rétrovirus endogènes; certaines d'entre elles sont utilisées comme modèles animaux de maladies humaines (diabète insulino-dépendant, lupus érythémateux disséminé).
(5) des taux significatifs d'anticorps anti-rétrovirus endogènes ont été décrits dans le cadre de maladies auto-immunes, systémiques ou inflammatoires; d'autres données dans ce sens ont été communiquées par plusieurs auteurs au IVème meeting européen sur les rétrovirus endogènes (Uppsala, octobre 1996). D'après Venables (communiqués du IVème meeting européen sur les rétrovirus endogènes, Uppsala, octobre 1996), on retrouve un taux d'anticorps anti-HERV-H' significativement élevé pendant la grossesse mais aussi dans le cadre de divers désordres auto-immuns tels que le syndrome de Sjögren, le lupus érythémateux disséminé ou la polyarthrite rhumatoïde, sans toutefois qu'une preuve de son implication directe puisse être apportée à ce jour.

L'implication des rétrovirus dans le phénomène d'auto-immunité reste compatible avec le caractère multifactoriel des maladies auto-immunes, systémiques ou inflammatoires qui confrontent des facteurs génétiques, hormonaux, environnementaux et infectieux.

Les particules observées dans les surnageants de cultures cellulaires de patients atteints de sclérose en plaques (Perron et coll, Res. Virol. 1989; 140: 551-561 /Lancet 1991; 337: 862-863 / Res. Virol. 1992; 143: 337-350) ou de polyarthrite rhumatoïde (données non publiées) peuvent résulter de l'expression: (i) d'un rétrovirus endogène compétent pour la réplication, (ii) de plusieurs rétrovirus endogènes défectifs coopérant par un phénomène de transcomplémentation ou (iii) d'un rétrovirus exogène.

Toutes ces observations permettent d'utiliser et considérer les matériels biologiques précédemment décrits, comme marqueur de la sclérose en plaques.

En particulier, les techniques de marquage suivantes sont considérées :
- balayage du génome humain avec des sondes d'hybridation à forte stringence, dérivées du matériel nucléique précédemment décrit,
- amplification directe d'ADN génomique par PCR, en utilisant des amorces spécifiques pour la région considérée
- analyse des régions flanquantes de gènes cellulaires étrangers.

## Revendications

1. Matériel, nucléique génomique rétroviral endogène HFRV-W, à l'état isolé ou purifié, comprenant une séquence nucléotidique choisie parmi SEQ ID NOs: 1 à 15 et leurs séquences complémentaires

2. Sonde nucléique de détection d'un matériel nucléique salon la revendication 1, **caractérisée en ce qu'**elle est choisie parmi SEQ ID NO : 16-18 et 21-28.

3. Sonde selon la revendication 2, **caractérisée en ce qu'**elle comprend un marqueur.

4. Amorce nucléique pour l'amplification par polymérisation d'un matériel nucléique selon la revendication 1, **caractérisée en ce qu'**elle est choisie parmi SEQ ID NO : 16-18 et 21-28.

5. Peptide codé par tout cadre de lecture ouvert de le séquence SEQ ID NO : 11 codant pour une ou des protéines ENV rétrovirales.

6. Peptide selon la revendication 5, **caractérisé en ce qu'**il forme un déterminant antigénique reconnu par des sera de patients affectés par la sclérose en plaques.

7. Utilisation d'un matériel nucléique selon la revendication 1, ou d'un peptide selon la revendication 5 ou 6, comme marqueur moléculaire de la sclérose en plaques, à l'exclusion de toute méthode de diagnostic appliquée au corps humain ou animal.

8. Utilisation d'un matériel nucléique selon la revendication 1, comme marqueur chromosomique d'une susceptibilité à la sclérose en plaques, à l'exclusion de toute méthode de diagnostic appliquée au corps humain ou animal.

9. Utilisation d'un matériel nucléique selon la revendication 1, comme marqueur de proximité d'un gène de susceptibilité à la sclérose en plaques, à 1 exclusion de toute méthode de diagnostic appliquée au corps humain ou animal.

## Claims

1. HERV-W endogenous retroviral genomic nucleic material, in the isolated or purified state, comprising a nucleotide sequence chosen from SEQ ID Nos. 1 to 15 and the sequences complementary thereto.

2. Nucleic probe for detecting a nucleic material according to Claim 1, **characterized in that** it is chosen from SEQ ID Nos. 16-18 and 21-28.

3. Probe according to Claim 2, **characterized in that** it comprises a label.

4. Nucleic primer for the amplification by polymerization of a nucleic material according to Claim 1, **characterized in that** it is chosen from SEQ ID Nos. 16-18 and 21-28.

5. Peptide encoded by any open reading frame of the sequence SEQ ID No. 11 encoding one or more retroviral ENV proteins.

6. Peptide according to Claim 5, **characterized in that** it forms an antigenic determinant recognized by sera from patients suffering from multiple sclerosis.

7. Use of a nucleic material according to Claim 1, or of a peptide according to Claim 5 or 6, as a molecular marker for multiple sclerosis, with the exclusion of any method of diagnosis applied to the human or animal body.

8. Use of a nucleic material according to Claim 1, as a chromosomal marker for susceptibility to multiple sclerosis, with the exclusion of any method of diagnosis applied to the human or animal body.

9. Use of a nucleic material according to Claim 1, as a marker for the proximity of a gene for susceptibility to multiple sclerosis, with the exclusion of any method of diagnosis applied to the human or animal body.

## Patentansprüche

1. Endogenes retrovirales Genomnukleinelement HERV-W im isolierten oder gereinigten Zustand, umfassend eine Nukleotidsequenz, die unter SEQ ID NO_{S}: 1 bis 15 und ihren komplementären Sequenzen ausgewählt wird.

2. Nukleinsonde zur Erfassung eines Nukleinelements nach Anspruch 1, **dadurch gekennzeichnet, dass** sie unter SEQ ID NO: 16-18 und 21-28 ausgewählt wird.

3. Sonde nach Anspruch 2, **dadurch gekennzeichnet, dass** sie einen Marker umfasst.

4. Nukleinkern für die Verstärkung eines Nukleinelements nach Anspruch 1 durch Polymerisation, **dadurch gekennzeichnet, dass** sie unter SEQ ID NO: 16-18 und 21-28 ausgewählt wird.

5. Peptid, das durch jeden offenen Leserahmen der Sequenz SEQ ID NO: 11, die für eine oder mehrere retrovirale Proteine ENV codiert, codiert ist.

6. Peptid nach Anspruch 5, **dadurch gekennzeichnet, dass** es eine Antigendeterminante bildet, die von Sera von Patienten, die an multipler Sklerose leiden, erkannt wird.

7. Verwendung eines Nukleinelements nach Anspruch 1 oder eines Peptids nach Anspruch 5 oder 6 als Molekularmarker der multiplen Sklerose unter Ausschluss jeder Diagnosemethode, die am menschlichen oder tierischen Körper angewandt wird.

8. Verwendung eines Nukleinelements nach Anspruch 1 als Chromosomenmarker einer Prädisposition für multiple Sklerose unter Ausschluss jeder Diagnosemethode, die am menschlichen oder tierischen Körper angewandt wird.

9. Verwendung eines Nukleinelements nach Anspruch 1 als Marker für die Nähe eines Prädispositionsgens für multiple Sklerose unter Ausschluss jeder Diagnosemethode, die am menschlichen oder tierischen Körper angewandt wird.
